# EUROPEAN PATENT SPECIFICATION

(11) **EP 2 978 840 B1**
(45) Date of publication and mention of the grant of the patent: **30.09.2020**
(21) Application number: 14722738.3
(22) Date of filing: 26.03.2014
(51) Int. Cl.: C12N 5/071

(54) **A HUMAN BLOOD-BRAIN BARRIER MODEL DERIVED FROM STEM CELLS**
HUMANES BLUT-HIRN SCHRANKE MODELL AUS STAMZELLEN
MODELE DE BARRIERE HEMATO-ENCEPHALIQUE HUMAINE

(30) Priority: 26.03.2013 PT 10685513
(43) Date of publication of application: 03.02.2016
(73) Proprietor: BIOCANT - CENTRO DE INOVAÇÃO EM BIOTECNOLOGIA, 3060-197 Cantanhede (PT); University of Artois, 62030 Arras Cedex (FR)
(72) Inventor: DA SILVA FERREIRA, Lino, 3030-076 Coimbra (PT); SEVIN, Emmanuel, Bernard, 62300 Lens (FR); CECCHELLI, Roméo Paul, 59650 Villeneuve d'Ascq (FR); ADAY, Sezin, 3060-132 Cantanhede (PT)
(74) Representative: Patentree
(86) International application number: PCT/IB2014/060186
(87) International publication number: WO 2014/203087

(56) References cited:
- EP-A1- 1 964 915
- WO-A2-2011/159572
- ETHAN S LIPPMANN ET AL: "Derivation of blood-brain barrier endothelial cells from human pluripotent stem cells", NATURE BIOTECHNOLOGY, vol. 30, no. 8, 24 June 2012 (2012-06-24), pages 783-791, XP055101029, ISSN: 1087-0156, DOI: 10.1038/nbt.2247
- ETHAN S LIPPMANN ET AL: "Modeling the blood-brain barrier using stem cell sources", FLUIDS AND BARRIERS OF THE CNS, BIOMED CENTRAL LTD, LONDON, UK, vol. 10, no. 1, 10 January 2013 (2013-01-10), page 2, XP021140444, ISSN: 2045-8118, DOI: 10.1186/2045-8118-10-2
- ATSUKO KAMIICHI ET AL: "Establishment of a new conditionally immortalized cell line from human brain microvascular endothelial cells: A promising tool for human blood-brain barrier studies", BRAIN RESEARCH, vol. 1488, 1 December 2012 (2012-12-01), pages 113-122, XP055131029, ISSN: 0006-8993, DOI: 10.1016/j.brainres.2012.09.042
- ROMEO CECCHELLI ET AL: "A Stable and Reproducible Human Blood-Brain Barrier Model Derived from Hematopoietic Stem Cells", PLOS ONE, vol. 9, no. 6, 1 January 2014 (2014-01-01) , pages e99733-e99733, XP055131025, ISSN: 1932-6203, DOI: 10.1371/journal.pone.0099733

## Description

### Technical Field

The present disclosure relates to a method to generate a population of endothelial cells showing a brain endothelial cells-like phenotype.

The present disclosure further relates to a population of endothelial cells, said cells showing phenotype similar to brain endothelial cells.

The present disclosure also relates to a stable and reproducible *in vitro* human brain-blood barrier model.

### Background

Blood-brain barrier (BBB) models can provide a valuable tool for studying mechanistic aspects related to the transport of drugs at the brain, as well as biological and pathological processes related to the BBB (Cecchelli, R., et al. Modelling of the blood-brain barrier in drug discovery and development; Nat Rev Drug Discov 6, 650-661 (2007)). Although several *in vitro* models were established using murine and bovine cells, the establishment of a stable human BBB model is very important to account for differences between species (Cecchelli, R., et al. Modelling of the blood-brain barrier in drug discovery and development; Nat Rev Drug Discov 6, 650-661 (2007)). Primary human brain endothelial cells (hBECs) and immortalized human cells have been used as *in vitro* models; however, several issues prevent their general use including constraints in obtaining human tissue, loss of hBEC phenotype during immortalized cell culture, or lack of important tight junctions and low TEER values as shown in human cell lines (Weksler, B.B., et al. Blood-brain barrier-specific properties of a human adult brain endothelial cell line. FASEB J 19, 1872-1874 (2005);Sano, Y., et al. Establishment of a new conditionally immortalized human brain microvascular endothelial cell line retaining an in vivo blood-brain barrier function. J Cell Physiol 225, 519-528 (2010)). Recently, hBECs have been differentiated from induced pluripotent stem cells (iPSCs); however, the low stability and high variability in the BBB system formed by different iPSC lines might preclude its general use (Lippmann, E.S., et al. Derivation of blood-brain barrier endothelial cells from human pluripotent stem cells. Nat Biotechnol (2012)).

For example, document WO/2006/056879 A1 relates to an immortalized human brain endothelial cell line that is useful as an *in vitro* model of the blood brain barrier. The document describes the generation of an immortalized human brain endothelial cell line (hCMEC/D3). The cell line was derived from primary brain endothelial cells transfected with a lentiviral vector system leading to the production of telomerase and SV40 Large T antigen. The cell line retained morphological characteristics of primary brain endothelial cells and expressed specific brain endothelial markers and cell surface adhesion molecules. Furthermore, the cell line expressed chemokine receptors and ATP binding cassette (ABC) - transporters. However, the expression level of ABCA2, MDR1, MRP4, BCRP, GUT1, 4F2hc, MCT1 and insulin receptor is 4 times lower in hCMEC/D3 cell line than in human brain microvessels (Ohtsoki et al., Molecular Pharmaceutics 2013, 10(1), 289-296). Furthermore, the cells show deficiency in typical and important brain endothelial properties such as low TEER value and relative high permeability towards small tracer molecules indicating paracellular leakiness and suboptimal formation of tight junctions.

Document WO/2007/072953 A1 intends to provide a screening system for a drug which passes the blood-brain barrier and acts on the center, a drug which acts on the blood-brain barrier per se, or a drug which is not expected to act in the center but migrates into the brain. This *in vitro* BBB model is formed by a co-culture of primary brain endothelial cells, pericytes and astrocytes in a three-dimensional culture device. The invention is not related to the use of human brain endothelial cells and therefore does not take into account with inter-species differences in terms of metabolism and physiology. Furthermore, even if a human BBB model could be proposed, it requires the isolation of human brain endothelial cells from an autopsy tissue or freshly resected brain specimens derived from brain tumor or epilepsy patients. The issue here is that brain endotheial cells are not available in enough number for this purpose, and do not have the enough stability to act as a reliable *in vitro* BBB model.

Document US/2012/0015395 A1 describes a method for producing brain specific endothelial cells, preferably comprising the steps of growing human pluripotent stem cells inducing differentiation of the cells by culturing the cells in unconditioned medium, and further expanding the endothelial cells in endothelial cell medium, wherein the expanded cells are GLUT-1⁺, PECAM-1⁺, claudin-5⁺, occludin⁺, ZO-1⁺, and p-glycoprotein⁺. The invention also claimed a method comprising the step of co-culturing the cells with a cell type selected from the group of astrocytes, neural progenitor cells, and pericytes. The brain endothelial cells derived from human pluripotent stem cells yielded TEER with an average value of 860 ± 260 Ω cm2 (Lippmann et. al., Nature Biotechnology 2012). Yet, the TEER values fluctuated over time. For example, the TEER values in a co-culture of brain endothelial cells with astrocytes changed 200% during the first 50 h. Furthermore, it is unclear the stability of the system overtime and its reproducibility.

Document WO/2007/140340 A2 related to methods for providing CD34+ cells from embryoid bodies and stimulating these cells to give rise to endothelial-like and/or smooth muscle-like cells. However, the object of the invention was not linked to the specification of the endothelial cells into brain endothelial cells.

### Summary

### Definitions

"CD34⁺ cells" refers to cells expressing CD34 antigen. This antigen is a single-chain transmembrane glycoprotein expressed in several cells including human hematopoietic stem and progenitor cells, vascular endothelial cells, embryonic fibroblasts and some cells in fetal and adult nervous tissue.
"Brain-like endothelial cells" refers to cells that share properties (gene, protein and functional) of fully functional brain endothelial cells, including the expression of at least one of the following markers, low density lipoprotein receptor, insulin receptor, leptin receptor, transferrin receptor, receptor for advanced glycation endproducts, retinol binding protein, SLC7A5, SLC2A1, SLC38A5, SLC16A1, ABCB1, ABCG2, ABCC1, ABCC2, ABCC4, ABCC5, claudin 1, claudin 3, ZO-1, occludin, JAM-A and claudin-5.
"Hematopoietic stem cells" refers to cells that can themselves or whose progeny can form myeloid, erythroid, and/or megakaryocyte colonies as described in Eaves, et al., Atlas of Human Hematopoietic Colonies, 1995, StemCell Technologies, Vancouver; Coutinho, et al, in Hematopoiesis: A Practical Approach, Testa, et al, eds., 1993, Oxford Univ. Press, NY, pp 75-106, and Kaufman, et al., PNAS, 2001, 98:10716-10721.
"Pericytes" refers to cells that express one of the following markers: vimentin, neuro-glial 2 (NG2), platelet-derived growth factor receptor beta (PDGFR-β), α-smooth muscle actin (a-SMA), cells that express one of the following markers: viet al., Current Neurovascular Research 2011, Modelling the neurovascular unit and the BBB with the unique function of Pericytes).

In view of the drawbacks to the pior art, one of the problems was to develop an *in vitro* human BBB system that could be stable for more than 15 days and be reproducible for different stem cells and could be implemented in different laboratories. The system described is the first human *in vitro* BBB system with high reproducibility - evaluated in three different laboratories; and in stem cells collected from more than 4 different donors - and stable - more than 20 days. This has not ever been addressed by previous technologies described in the literature.

An aspect of disclosed subject matter relates to human brain-like endothelial cells wherein at least a portion of the cells express at least one of the following markers: ZO-1, occludin, JAM-A, claudin-5, claudin-3, claudin-1, preferably express ZO-1 and claudin-1.

In embodiments of the disclosure, the brain-like endothelial cells further express at least one of the following transporters or receptors: aminoacid - SLC7A5, SLC16A1, glucose - SLC2A1.

In embodiments of the disclosure, the brain-like endothelial cells further express a portion of at least one of the following molecules: CD40, VCAM-1.

Others embodiments of the disclosure, at least a portion of the brain-like endothelial cells express at least one of the following transcripts of key efflux transporters as P-glycoprotein, breast cancer resistance protein and multidrug resistance protein.

In others embodiments of the disclosure, at least a portion of the brain-like endothelial cells expresses at least one of the following genes up-regulated: SLC44A5, SLC25A27 the endothelial cells, SLC23A3.

In others embodiments of the disclosure, at least a portion of the brain-like endothelial cells further express at least one of the following markers: lipoprotein receptor, insulin receptor, leptin receptor, transferrin receptor, receptor for advanced glycation endproducts, retinol binding protein,SLC38A5, ABCB1, ABCG2, ABCC1, ABCC2, ABCC4, ABCC5.

In others embodiments of the disclosure, endothelial cells derived from stem cells, can be preferably from hematopoietic stem/progenitor cells (CD34⁺ cells) derived from human cord blood, but can be extended to endothelial cells derived from hematopoietic stem/progenitor cells derived from human peripheral blood.

A further aspect of the disclosure relates to a method of inducing a blood brain barrier phenotype in endothelial cells derived from stem cells, preferably from hematopoietic stem/progenitor cells (CD34⁺ cells) derived from human cord blood, but can be extended to endothelial cells derived from hematopoietic stem/progenitor cells derived from human peripheral blood.

An aspect of disclosed subject matter relates to a method for obtaining *in vitro* human brain-like endothelial cells comprising the following steps:
- contacting a population of stem cells with a differentiation medium to form endothelial cells;
- co-culturing the said endothelial cells with pericytes or with cells of the neurovascular unit or with a pericytes conditioned medium, to obtain brain like endothelial cells, preferably during at least 4 days, more preferably during 5-7 days, namely 5,6,7 days.

In others embodiments of the method for obtaining *in vitro* human brain-like endothelial cells wherein the said stem cells may be CD34+ derived from human cord blood, or cells from human peripheral blood. Further aspects of the disclosure relate to a method for obtaining *in vitro* human brain-like endothelial cells wherein the cells are grown on solid support, preferably transwell systems or well plates. In preferred embodiments, the pericytes can be placed in the bottom of each plate. In others embodiments of the method for obtaining *in vitro* human brain-like endothelial the differentiation medium may be EGM-2 medium with 20% (v/v) FBS and 50 ng/mL of VEGF165. In others embodiments of the method for obtaining *in vitro* human brain-like endothelial cells the pericytes may express at least one of the following markers: vimentin, PDGF-β, NG-2, α-SMA; γ-GT. Further aspects of the disclosure relate to a method for obtaining *in vitro* human brain-like endothelial cells the pericytes may be seeded at a density of 40x10³- 50x10³, preferably 45x10³ cells.

In others embodiments of the method, the pericytes might be replaced by a cell line that secrete Wnt3a or Wnt7a.

In others embodiments of the method for obtaining *in vitro* human brain-like endothelial cells the pericytes - conditioned medium (obtained from 45 x10³ cells of pericytes cultured for 6 days in a well of a 12-well plate) may be replaced every day.

A further aspect of disclosed subject matter relates to a method for evaluating blood-brain barrier permeability of a substance, cell or protein comprising exposing the said test substance, cell or protein to the *in vitro* endothelial cells, said substance can be any synthetic or natural compound. Further aspects of the disclosure relate to a method for evaluating blood-brain barrier permeability of a substance, cell or protein by the measurement of efflux transport, preferably in the presence or absence of inhibitors of the efflux pumps. Preferably, the inhibitors may be at least one of the following: cyclosporin-A, PSC-833, MK-571, KO-143, verapamil or elacridar.

A further aspect of aspect of disclosed subject matter relates to a method for evaluating blood-brain barrier metabolism of a test substance, cell or protein which comprises the following steps:
- contacting a test substance, cell or protein to the brain endothelial cells previously described;
- analysing the metabolic degradation of the said test substance, cell or protein.
Further aspects of the disclosure relate to the method for evaluating blood-brain barrier toxicity of a test substance comprises the culturing the said brain endothelial cells in the presence of the said test substance.

The viability can be determined by a live/dead assay, preferably using calcein and propidium iodide as reagents, ATP production, cell membrane damage by the release of lactate dehydrogenase, cell replication by a BrdU assay. Any changes in the BBB functionality (e.g: permeability to a non permeant marker) *in vitro* could be used as an alternative toxicological endpoint. A further aspect of the disclosure relates to the method for evaluating blood-brain barrier metabolism of a test substance comprises the culturing the said brain endothelial cells in the presence of the said test substance.

A further aspect of the disclosure relates to a kit for measuring blood-brain barrier permeability of a substance, comprising the *in vitro* human endothelial cells previously described.

In various embodiments, pericytes are preferably derived from bovine, but they can be isolated from any other species. They are characterized by a set of different markers including PDGF-β in other species. They are characterized by a set of different markers including PDGF-β isolated from endothelial cell culture, α-smooth muscle actin (a-SMA), γ-glutamyl-transpeptidase and P-glycoprotein (P-gp) and others that someone skilled in the art will identify. So far, there is no single marker that differentiates pericytes from other cells. In one embodiment, the pericytes are placed in the bottom of the plate, but they can be also applied in one of the sides of the transwell filter. In a preferable embodiment, the pericytes are seeded at a density of 45×10³ cells into each well of 12-well plates. The cells are cultured on Dulbecco's Modified Eagle's Medium (DMEM) supplemented with 20% (v/v) fetal bovine serum (FBS), 2 mM L-glutamine, 50 µg/mL gentamycin and 1 ng/mL basic fibroblast growth factor.

In a preferred embodiment, the pericytes are cultured in the presence of the endothelial cells to induce BBB properties. Conditioned medium obtained from pericytes might have the same inductive effect on endothelial cells if medium is replaced every day, and at suitable concentrations.

In one embodiment, the BBB properties of endothelial cells can be achieved by supplementing the culture medium with Wnt3a, Wnt7a or a mixture of Wnt3a and Wnt7a. In a preferable embodiment, the concentration of Wnt3a and Wnt7a is 6.25 ng/mL.

### Brief description of drawings

Without intent to limit the disclosure herein, this application presents attached drawings of illustrated embodiments for an easier understanding.

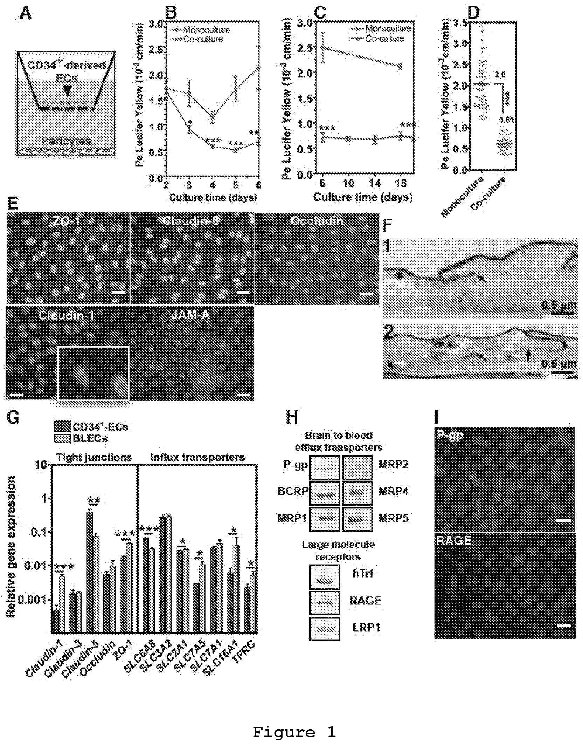

**Figure** 1: Expression of BBB markers, stability and functional properties of a monolayer of human BLECs;
   (A) BLECs were obtained by the co-culture of CD34+- derived ECs with pericytes for 6 days in a transwell™ system.
   (B-D) Paracellular permeability to lucifer yellow of EC monolayers either cultured alone or with pericytes. Results are Mean ± SEM (n≥4);
   (E) Expression of endothelial and BBB markers in BLECs as obtained by immunofluorescence;
   (F) Electron micrographs of ECs cultured alone (2,3) or with pericytes (1);
      (1) In the intercellular cleft, WGA-HRP penetrates from the luminal compartment (asterisks) to the tight junction, which occludes the cleft (arrows). From this point, the intercellular space is free of the electron-dense reaction product;
      (2) When ECs are cultured alone, there is no occlusion of the intercellular space between the ECs in 84% of the cases, and the tracer penetrates from the luminal compartment (asterisks) trough the entire intercellular cleft and is deposited in the underlying matrix (arrowheads);
   (G) BLEC gene expression of tight junctions and influx transporters. Results are Mean ± SEM (n=3);
   (H) BLEC gene expression of efflux transporters and large molecule receptors;
   (I) Expression of P-gp and RAGE as evaluated by immunofluorescence. In E and I, bar corresponds to 50 µm. *P<0.05, **P<0.01, ***P<0.001.

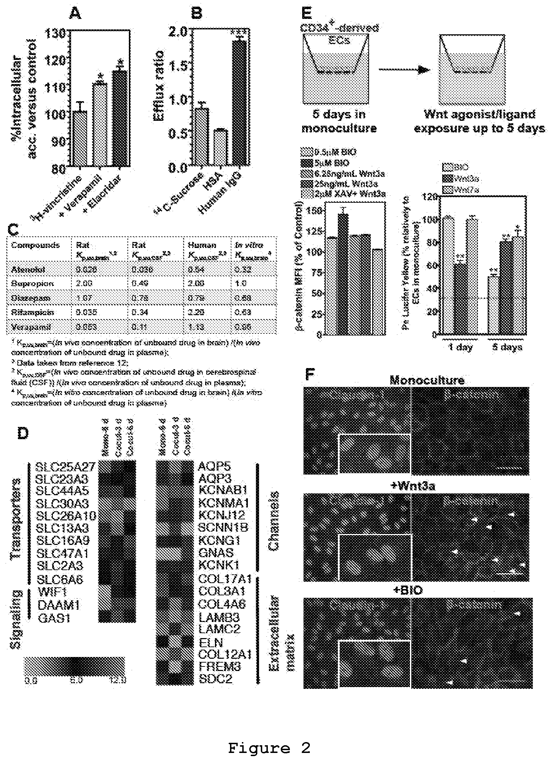

**Figure 2****:** Functional properties of BLECs and mechanism for the in the induction of BBB properties in CD34+-derived ECs;
   (A) Effect of P-gp protein inhibition on active transport of drugs;
   (B) Efflux ratio of small (sucrose) and large (HSA and IgG) molecules. In A and B: results ± SEM (n=3-7);
   (C) Unbound brain-to-plasma or CSF-to-plasma concentration ratio's for human and rat;
   (D) Expression of BBB markers as evaluated by whole genome microarrays of monocultures or co-cultures of CD34+-derived ECs with pericytes at day 3 and 6;
   (E) Effect of Wnt3a, Wnt7a and BIO in the expression of β-catenin (after 1 day) as well as in the paracellular permeability (at days 1 and 5) of monocultures of CD34+-derived ECs. Results are Mean ± SEM (n=3-6). The dashed line represents the paracellular permeability of ECs in co-culture with pericytes for 6 days. For permeability results the concentrations of Wnt3a, Wnt7a and BIO were 6.25 ng/mL, 6.25 ng/mL, and 0.5 µM;
   (F) Expression and localization of claudin-1 (at day 6) and total β-catenin (day 3) in monoculture of CD34+- derived ECs cultured in medium supplemented with BIO (0.5 µM) or Wnt3a (6.25 ng/mL). Arrow-heads indicate nuclear accumulation of β-catenin. Bar corresponds to 50 µm. *P<0.05, **P<0.01, ***P<0.001.

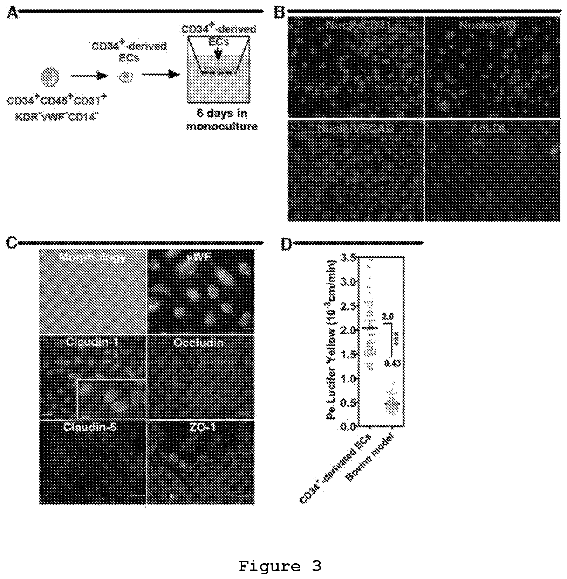

**Figure** 3: Differentiation of human umbilical cord CD34+ cells into ECs and evaluation of their paracellular permeability;
   (A) Schematic representation of the differentiation of hematopoietic stem cells (CD34+CD45+CD31+KDR-vWF-CD14-) into ECs (2-3 weeks of differentiation) and evaluation of their paracellular permeability (Pe) using a Transwell™ system;
   (B) ECs immediately after differentiation (before culture in the Transwell™ system) express typical EC markers including CD31, VE-cadherin (VECAD), vWF and are able to incorporate AcLDL;
   (C) ECs after culture in the Transwell™ system have typical cobblestone morphology, express vWF and markers associated to hBECs such as claudin-5, ZO-1, and occludin; however, the expression of all these markers is discontinuous and cells do not express claudin-1 at cell-cell contacts. Bar corresponds to 50 µm;
   (D) Paracellular permeability of human ECs in monoculture and bovine ECs in co-culture with astrocytes for 12 days.

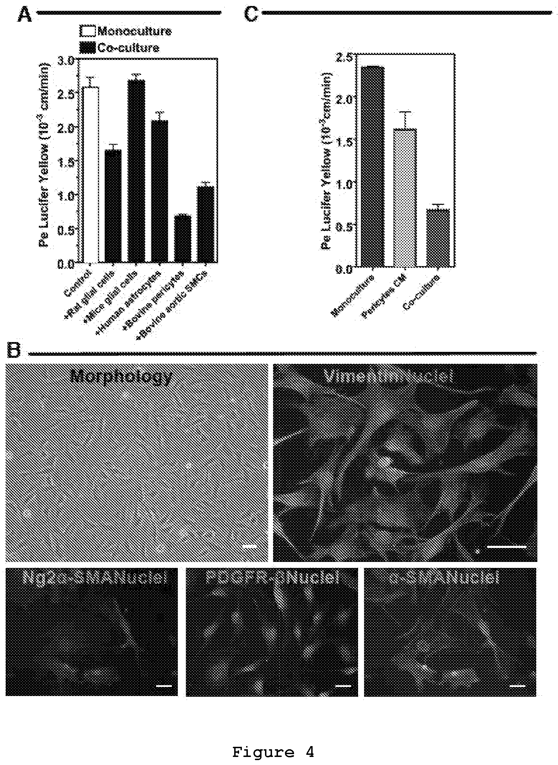

**Figure 4****:** (A) Paracellular permeability of CD34+-derived ECs after co-culture with different types of cells in EGM-2 supplemented with 2% fetal calf serum FCS). Results are Mean ± SEM (n=6) ; (B) Characterization of bovine pericytes by phase contrast and Immunocytochemistry for the expression of vimentin, neuro-glial 2 (NG2), platelet-derived growth factor receptor beta (PDGFR-β), and α-smooth muscle actin (α-SMA). Scale bar corresponds to 50 µm. CM stands for conditioned media;(C) The induction of BBB properties on CD34+-derived ECs requires the presence of pericytes in the co-culture system since pericyte-conditioned medium does not have the same BBB-inductive properties.
**Figure 5****:**
   (A) Double immunostaining for anti-human receptor for advanced glycation endproducts (RAGE) and anti-human organic cation/carnitine transporter (OCTN2; also known as SLC22A5) in monoculture(A.1) or in co-culture of CD34+-derived ECs with pericytes (A.2) at day 6. In the co-culture system, RAGE is present essentially in the luminal side of endothelial cells and OCTN2 in the abluminal side, while in mono-culture, both markers seem to be located in the same plane. Bar corresponds to 10 µm;
   (B) Paracellular permeability in a co-culture of CD34+-derived ECs with pericytes at day 6 obtained from different donors. Results are Mean ± SEM (n=4);
   (C) Interlaboratory reproducibility. The BBB was generated in two different laboratories. The paracellular permeability to lucifer yellow was not statistical significant. Results are Mean ± SEM (n≥4) ;
   (D) Stability of the BBB properties after removal of the pericytes. CD34+-derived ECs were in co-culture with pericytes for 14 days (1) or in co-culture for 6 days and then 8 days in monoculture (2).

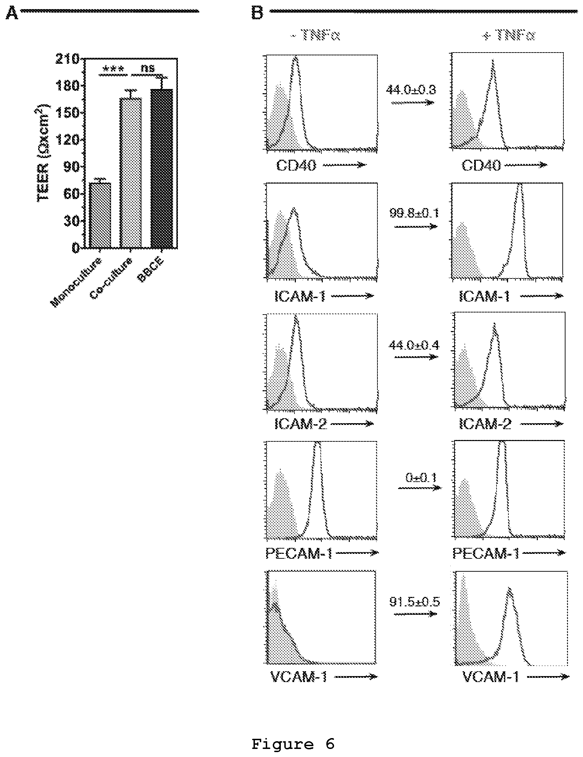

**Figure 6****:** (A) Transendothelial electrical resistance (TEER) of monocultures of CD34+-derived ECs or co-cultures of ECs with pericytes for 6 days.The TEER of the co-culture of ECs was compared with the gold standard of bovine brain microvascular endothelial cells co-cultured with bovine astrocytes for 12 days on insert filters 30 mm diameter. Values are Mean ± SEM, n=4. *** P<0.001; ns means P>0.05; (B) Expression of adhesion molecules by ECs in co-culture with pericytes. The expression of the adhesion molecules was assessed by flow cytometry analysis on untreated and treated ECs by TNFα (10 ng/mL) for 24 h.

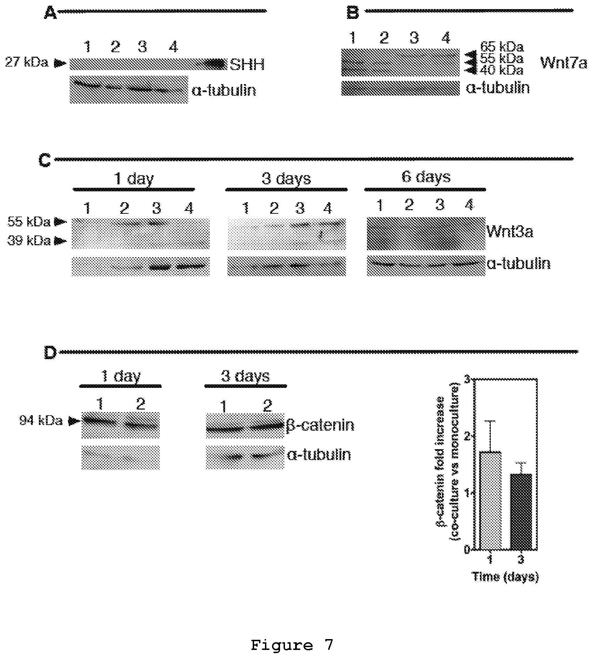

**Figure 7****:** (A-C) Western blot for the expression of Shh (A), Wnt7a (B), Wnt3a (C) and total β-catenin (D) in CD34+-derived ECs in monoculture (1) or in co-culture with pericytes (2), or pericytes in monoculture (3) or pericytes in co-culture with ECs (4), for 6 days. Human recombinant Wnt3a, Wnt7a and Shh were used as a positive control. Data shown are representative of n=2. In D: results ± SEM, n=2.

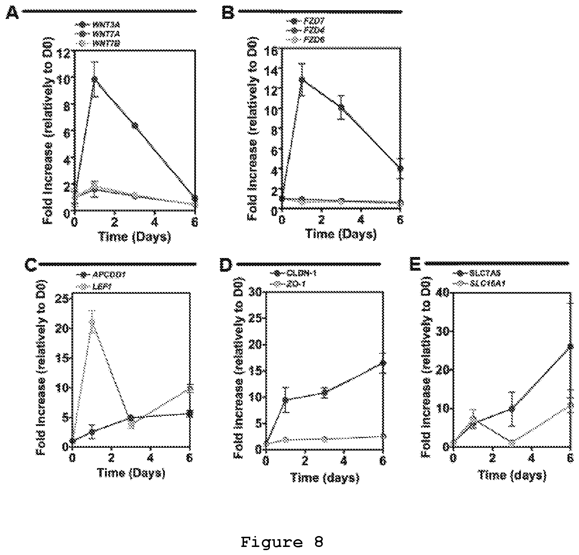

**Figure 8****:** qRT-PCR results showing changes on Wnt signaling (A-C), tight junctions (D) and BBB transporters (E) genes on CD34+-derived ECs co-cultured with pericytes for 1,3 and 6 days. Values are Mean ± SEM, n=4. Our results show that Wnt3a transcript increased significantly at day 1 followed by a decrease at day 6 to baseline levels. Genes of canonical Wnt ligands Wnt7a and Wnt7b, which have been reported to be involved in BBB development, increased slightly at day 1 and then decreased at day 3 to baseline levels. The expression of genes encoding Wnt receptor frizzled 4 (FZD4) and frizzled 6 (FZD6) were not affected by the co-culture system; however, Wnt receptor frizzled 7 (FZD7) was significantly up-regulated up to 6 days. The expression of LEF1, the β-catenin-associated transcription factor, peaked at day 1 matching the profile observed for Wnt3a and FZD7. The expression of APCDD1, an antagonist of Wnt signaling and highly expressed in adult brain endothelial cells, peaked at day 3, at the time that Wnt3a drops significantly. Finally, genes related to tight junctions such as claudin 1 and ZO-1 and the transporters SLC7A5 and SLC16A1 are upregulated overtime.

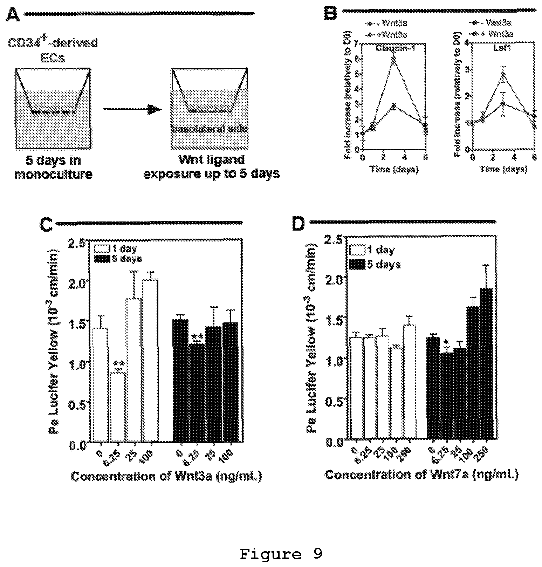

**Figure 9****:** Modulation of Wnt signaling activates the barrier properties of ECs in monoculture;
   (A) Schematic representation of the methodology used to assess the modulation of Wnt signaling. CD34+-derived ECs were seeded in a transwell™ insert coated with Matrigel at a density of 80,000 cells. Wnt ligands were added in the culture medium at the basolateral side;
   (B) qRT-PCR results showing differences in expression of claudin-1 and Lef1 genes on CD34+-derived ECs cultured with or without Wnt3a. Values are Mean ± SEM, n=4;
   (C-D) Paracellular permeability of untreated ECs or ECs treated with different concentrations of human recombinant protein Wnt3a (C) or Wnt7a (D) for 5 days. Results are Mean ± SEM (n=4).

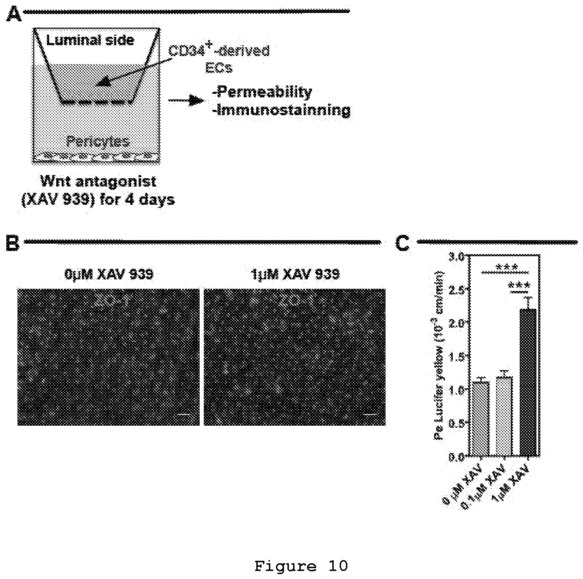

**Figure 10****:** Abrogation of Wnt signaling in ECs during co-culture with pericytes affect their paracellular permeability;
   (A) Schematic representation of the methodology used to assess the effect of abrogation of Wnt signaling. CD34+-derived ECs were seeded in a transwell™ insert coated with Matrigel at a density of 80,000 cells and cultured in medium supplemented with XAV 939 (0.1 and 1 µM). In the bottom of the transwell™ was seeded 45,000 bovine pericytes. After 4 days of coculture, the paracellular permeability and cell organization were evaluated;
   (B) Fluorescence microscopy images showing the expression of ZO-1 in untreated ECs or ECs treated with XAV 939 (1 µM) for 4 days;
   (C) Paracellular permeability of untreated ECs or ECs treated with 0.1 or 1 µM XAV939 for 4 days. Results are Mean ± SEM (n=4).

### Detailed description

In the present disclosure is described a method to generate a human blood-brain barrier model using cord blood-derived hematopoietic stem cells. The cells were initially differentiated into endothelial cells followed by the induction of blood-brain barrier (BBB) properties by co-culture with pericytes. The brain-like endothelial cells (BLECs) express tight junctions and transporters typically observed in brain endothelium and maintain expression of most in vivo BBB properties for at least 20 days.

To differentiate stem cells into endothelial cells, CD34+CD45+CD31+KDR-vWF-CD14- cells isolated from cord blood were initially cultured for 15-20 days in EGM-2 medium with 20% (v/v) FBS and 50 ng/mL of VEGF165 (Supplementary Fig. 1A). At this stage, cells have a cobblestone-like morphology and express high levels of endothelial cells markers, including CD31, VE-cadherin and vWF (Supplementary Fig. 1B). When these cells were grown to confluence on filters for 6 days they show discontinuous expression of ZO-1, occludin and claudin-5, do not express claudin-1 at cell-cell contacts and have high permeability to Lucifer yellow (2.0 × 10-3 cm/min) as compared to bovine BECs (Supplementary Figs. 1C and 1D).

To induce BBB properties in CD34+-derived endothelial cells, cells were seeded in a transwell™ system and co-cultured with pericytes (Fig. 1A). Pericytes were selected after a screening of different cell types from the neurovascular unit (Supplementary Figs. 2A and 2B) and because of their role in the stabilization/maturation of BBB (Armulik, A., et al. Pericytes regulate the blood-brain barrier. Nature 468, 557-561 (2010); Daneman, R., Zhou, L., Kebede, A.A. & Barres, B.A. Pericytes are required for blood-brain barrier integrity during embryogenesis. Nature 468, 562-566 (2010)). Under these conditions, the permeability of endothelial cells decreases during the first 3 days until it reaches a stationary phase at day 4 (Fig. 1B), maintaining its stability up to 20 days (Fig. 1C). At day 6, the cells had low permeability values (0.61 × 10-3 cm/min) similarly to the values found in other BBB models (Deli, M.A., et al. Permeability studies on in vitro blood-brain barrier models: physiology, pathology, and pharmacology. Cell Mol Neurobiol 25, 59-127 (2005)) (Fig. 1D), they showed a continuous expression of ZO-1, occludin, JAM-A and claudin-5 at cell-cell contacts (Fig. 1E) and they were able to block the passage of wheat germ agglutinin (WGA)- horseradish peroxidase (HRP) in contrast with monolayers of CD34+-derived endothelial cells where WGA-HRP reached the underlying matrix (Fig. 1F). Importantly, the induction of BBB properties in CD34+-derived endothelial cells is highly reproducible since similar permeability results were obtained for cells derived from multiple human donors (Supplementary Fig. 3B) and in 3 different laboratories (Supplementary Fig. 3C). Furthermore, the BBB properties of CD34+-derived endothelial cells are lost if the pericytes are removed from the co-culture system (Supplementary Fig. 3D) showing that the crosstalk between the two cells is important to maintain the BBB properties. Cells co-cultured with pericytes for 6 days express transcripts encoding tight junctions such as ZO-1 and claudin-1 higher than in endothelial cells in monoculture, while the expression of claudin-3 and occludin was similar (Fig. 1G). Importantly, the expression of influx transporters, specifically the expression of aminoacid (SLC7A5, SLC16A1) and glucose (SLC2A1) transporters and receptors (e.g. transferrin receptor; TFRC) was increased when the cells were co-cultured with pericytes relatively to cells cultured alone. In addition, endothelial cells co-cultured with pericytes for 6 days express transcripts of key efflux transporters such as P-glycoprotein (P-gp), breast cancer resistance protein (BCRP) and multidrug resistance protein (MRP; subfamily of the ATP-binding cassette (ABC) transporters) family (Fig. 1H). As in hBECs, the receptor for advanced glycation end products (RAGE) and P-gp protein were expressed as confirmed by immunofluorescence (Fig. 1I), being RAGE located at the luminal side of cells (Supplementary Fig. 3A). Overall, endothelial cells co-cultured with pericytes for 6 days have BBB properties at gene, protein and permeability levels, and from now on are named as brain endothelial-like cells (BLECs).

BLECs have the ability to act as an active barrier. The inhibition of P-gp protein by verapamil or elacridar, and the concomitant blocking of the active transport of drugs to outside the cell, it leads to a significant increase in the accumulation of the antitumor drug vincristine (Fig. 2A). This result demonstrates that P-gp is functionally active in BLECs. The higher efflux ratio of IgG as compared to human serum albumin shows receptor-mediated transport of macromolecules across the polarized monolayer (Fig. 2B). In addition, BLECs have the ability to form a monolayer that has a transendothelial electric resistance (TEER) similar to monolayers of bovine BECs (Supplementary Fig. 4A) and higher than monolayers of human hCMEC/D3 cell line (<40 Ωcm2) (Weksler, B.B., et al. Blood-brain barrier-specific properties of a human adult brain endothelial cell line. FASEB J 19, 1872-1874 (2005)). Moreover, BLECs express constitutively the adhesion molecule ICAM-2, typically found in hBECs (Bo, L., et al. Distribution of immunoglobulin superfamily members ICAM-1, -2, -3, and the beta 2 integrin LFA-1 in multiple sclerosis lesions. J Neuropathol Exp Neurol 55, 1060-1072 (1996)), and show an up-regulation in the expression of ICAM-1, ICAM-2, CD40 and VECAM-1 after stimulation with 10 ng/mL TNF-α for 24 h, as hBECs (Weksler, B.B., et al. Blood-brain barrier-specific properties of a human adult brain endothelial cell line. FASEB J 19, 1872-1874 (2005) (Supplementary Fig. 4B). Finally, the *in vitro* ratio of concentrations of unbound drug in brain and plasma for atenolol, bupropion, rifampicin and verapamil were closer to the *in vivo* ratio of concentrations of unbound drugs in cerebrospinal fluid (CSF) and plasma reported in humans than in rats(Friden, M., Gupta, A., Antonsson, M., Bredberg, U. & Hammarlund-Udenaes, M. In vitro methods for estimating unbound drug concentrations in the brain interstitial and intracellular fluids. Drug Metab Dispos 35, 1711-1719 (2007)) (Fig. 2C). Together, these results indicate that brain-like endothelial cells can be used to predict accurately in humans the in vivo transport of drugs with different properties.

To study the induction of BBB properties in CD34+-derived endothelial cells, these cells cultured alone or with pericytes for 3 or 6 days were characterized by whole genome microarrays. Gene expression analyses at 6 days show that 84 and 2 genes are up- and down-regulated in CD34+-derived endothelial cells in co-culture, respectively, relatively to CD34+-derived endothelial cells in monoculture (Supplementary Tables 3 and 4). From the overall up-regulated genes, 3 genes were related with influx transporters including SLC44A5, SLC25A27 and SLC23A3, and 2 genes were related with Wnt signaling (Wnt inhibitory factor 1 and disheveled associated activator of morphogenesis (Cecchelli, R., et al. Modelling of the blood-brain barrier in drug discovery and development. Nat Rev Drug Discov 6, 650-661 (2007)) (Daam 1)) (Fig. 2D). Yet, the expression of most markers associated to BBB (tight junctions and transporters) was not significantly different in CD34+-derived endothelial cells in monoculture and co-culture, which indicates that pericytes exert a discrete influence on endothelial BBB-specific genes. Gene expression on CD34+-derived endothelial cells in co-culture at day 6 and 3 was significantly different regarding BBB markers, specifically for efflux transporters including solute carrier family members SLC2A3, SLC6A6 and SLC47A1 (downregulated at day 6), and members SLC30A3, SLC26A10, SLC13A3 and SLC44A5 (upregulated at day 6) and non-BBB markers such as channels and extracellular matrix (Supplementary Tables 3 and 4). Together these results show that the induction process is a dynamic process affecting the expression of transporters, channels and extracellular matrix components.

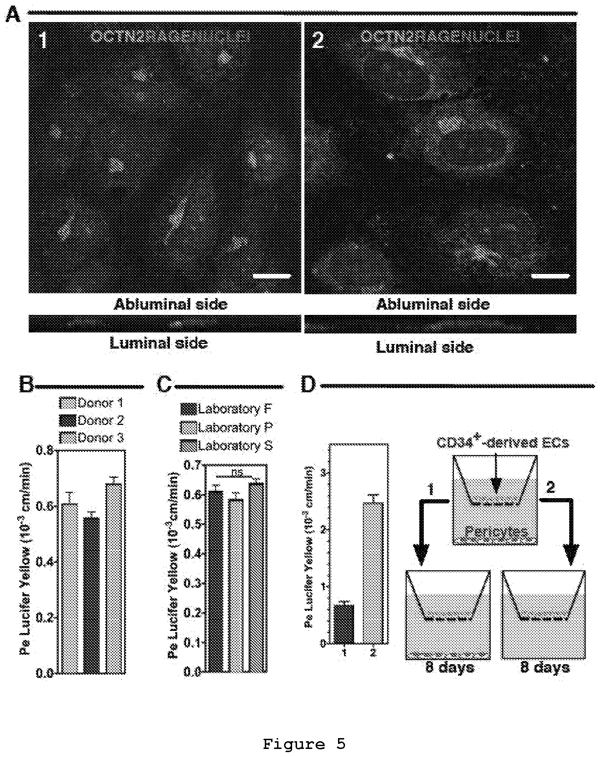

Two major pathways regulating the formation of BBB are the canonical Wnt/wingless pathway acting via β-catenin stabilization and Sonic hedgehog (Shh) pathway (Liebner, S., et al. Wnt/beta-catenin signaling controls development of the blood-brain barrier. J Cell Biol 183, 409-417 (2008); Alvarez, J.I., et al. The Hedgehog pathway promotes blood-brain barrier integrity and CNS immune quiescence. Science 334, 1727-1731 (2011); Daneman, R., et al. Wnt/beta-catenin signaling is required for CNS, but not non-CNS, angiogenesis. Proc Natl Acad Sci USA 106, 641-646 (2009). Protein analyses show that pericytes do not express Shh but do express Wnt ligands such as Wnt3a and Wnt7a (Supplementary Fig. 5). By other hand, endothelial cells express at gene level Wnt receptors such as frizzled receptor 4, 6 and 7 (FZD4, FZD6 and FZD7), and in co-culture with pericytes, they show an up-regulation in the expression of Wnt3a and FZD7 receptor during the first day followed by a decrease in the next 5 days (Supplementary Fig. 6). This was accompanied by an increase of APCDD1, an antagonist of Wnt signaling, that peaked at day 3, and an increase of the tight junctions ZO-1 and claudin-1 (Supplementary Fig. 6). To determine whether the activation of Wnt is required for the induction of barrier properties in CD34+-derived endothelial cells, these cells were cultured alone for 5 days and then exposed them to Wnt ligands/agonists. Endothelial cells respond rapidly to BIO, a specific pharmacological inhibitor of glycogen synthase kinase-3 (GSK-3) and thus an activator of Wnt signaling, or Wnt3a by increasing the expression of active β-catenin (Fig. 2E). The paracellular permeability of Wnt3a-treated endothelial cells to Lucifer Yellow was statistical lower (P<0.01, n=4) for short-term (1 day) and long-term (5 days) as compared to untreated cells (Fig. 2E and Supplementary Fig. 7). The effect of Wnt7a and BIO was only observed at day 5. During the induction process by Wnt 3a or BIO, there is an increase in the expression and nuclear localization of total β-catenin (Fig. 2F and Supplementary Fig. 5) and the localization of claudin-1 at the cell-cell contacts (Fig. 2F). The localization of claudin-1 at the periphery of the cells might explain the restrictive permeability of endothelial cells in co-culture with pericytes. Overall, results indicate that Wnt pathway contributes, at least in part, for the induction of BBB properties in CD34+-derived endothelial cells.

To further confirm the role of Wnt pathway in the induction of BBB properties, was abrogated the Wnt signaling in endothelial cells co-cultured with pericytes. Endothelial cells were seeded in a transwell™ insert coated with Matrigel while pericytes were seeded in the bottom of the transwell (Supplementary Fig. 8). Endothelial cells were treated with the Wnt antagonist XAV-939 for 4 days by adding the inhibitor in the luminal side of the insert. The abrogation of Wnt pathway, in conditions that did not affect cell viability, increased the paracellular permeability of the endothelial monolayer to lucifer yellow. These results again indicate that Wnt signaling is required for the BBB properties in CD34+-derived endothelial cells co-cultured with pericytes.

In summary, was generated a human *in vitro* BBB model from endothelial cells derived from cord blood hematopoietic stem cells that is highly reproducible and stable for at least 20 days after its derivation. Is provided *in vitro* evidence for a role of pericytes in the induction of BBB formation through the canonical Wnt pathway. Due to the relative easy access to cord blood stem cells, this model can be adopted by the research community to improve the delivery of therapeutic agents into the central nervous compartment for the treatment of stroke, multiple sclerosis and brain tumors.

In one embodiment of the present disclosure can be used as a method to measure BBB permeability to a test substance. The test substance may be any synthetic or natural compound, with variable molecular weight and hydrophilicity/hydrophobicity ratio. The method of the disclosure can measure passive diffusion or active transport, as appreciated by those skilled in the art. Efflux transport can be measured wherein measuring permeability values is performed in the presence or absence of inhibitors of the efflux pumps such as, but not limited to, cyclosporin-A, PSC-833, MK-571, KO-143. The methods of the present disclosure can also be used to measure blood brain barrier metabolism of a substance by measuring permeability values and profiling the metabolic degradation of compounds of interest as a function of time using quantitative analytical techniques such as high pressure liquid chromatography and mass spectrometry. Test substances that prove to pass our BBB *in vitro* model may be further analyzed for their pharmacological profile.

In another embodiment, the *in vitro* BBB model of the present disclosure may be useful as a method for determining the toxicity of a test substance or vector towards the BBB. In this case, the method comprises the culture of the brain endothelial-like cells in the presence of the test substance and assessing its viability after a certain time. A range of concentrations of the test substance can be used to determine the IC50. Cell viability can be determined by a live/dead assay using calcein and propidium iodide as reagents, ATP production, cell membrane damage by the release of lactate dehydrogenase, cell replication by a BrdU assay.

In another embodiment, the *in vitro* BBB model can be used to design more effective vectors to target or delivery drugs into the brain. This might be useful for the treatment of vascular dysfunction in patients with Alzheimer's. Neudegeneration is likely a consequence of altered drug transport across the BBB and abnormal cerebral blood flow due to amyloid peptide deposition. Our *in vitro* BBB model can be very useful for testing drug candidates for the treatment of Alzheimer.

### Isolation and differentiation of CD34+ cells from UCB

In a preferred embodiment CD34+ cells may be isolated from human umbilical cord blood and differentiated into endothelial cells according to a protocol previously reported by us (Pedroso, D.C., et al. Improved survival, vascular differentiation and wound healing potential of stem cells co-cultured with endothelial cells. PLoS One 6, e16114 (2011)) Briefly, isolated CD34+ cells were cultured in EGM-2 medium (preferably Lonza) supplemented with 20% (v/v) fetal bovine serum (preferably FBS; Life Technologies) and 50 ng/mL of VEGF165 (preferably PeproTech Inc.), on 1% gelatin-coated 24-well plates (2x10⁵ cells/well). After 15-20 days endothelial cells are seen in the culture dish. For each experiment, the cells were expanded in 1% (w/v) gelatin-coated 100 mm Petri dishes (preferably BD Falcon) in EGM-2 medium (with all the supplements except FBS and gentamycin/amphotericin) supplemented with 2% (v/v) FBS, 50 µ/mL gentamycin (preferably Biochrom AG) and 1 ng/mL home-made bFGF.

### Differenciation of CD34+ derived endothelial cells into brain like endothelial cells by pericytes

### Isolation of pericytes

In a preferred embodiment pericytes may be extracted from freshly collected bovine brain capillaries. Brain capillaries were collected on a 60 µm nylon sieve (preferably Blutex®, Saati, France) as described by Méresse et al. (1989) and suspended in Hanks Balanced Salt Solution (preferably HBSS, Sigma-Aldrich) containing 10 mM HEPES and 0.1% BSA. This suspension was centrifuged at 1000g for 7 min at room temperature. The pellet was then digested with 2 mg/mL collagenase-dispase (preferably Roche Diagnostics), 10 µ/mL DNaseI (preferably Roche Diagnostics) and 0.147 µ/mL TLCK (preferably Sigma-Aldrich), for 30 minutes at 37°C in a shaking water bath. After washes, the digested capillaries were seeded onto growth factor reduced Matrigel (preferably BD Biosciences) -coated dishes (preferably Corning) containing pericyte growth culture medium: DMEM (preferably Life Technologies) supplemented with 20% fetal calf serum (preferably Integro), 2 mM L-glutamine (preferably Merck Chemicals), 50 µ/mL gentamicin (preferably Biochrom AG) and 1 ng/mL bFGF (preferably Sigma-Aldrich). The medium was changed every other day. Pericytes and endothelial cells migrated from the vessels walls. Pericytes rapidly overgrew from capillaries and invaded the whole surface of the dishes. Confluent cultures consisting almost exclusively of pericytes, were dissociated using trypsin/EDTA saline solution (preferably 0.05%/0.02% Biochrom AG), and cells were frozen in liquid nitrogen. For experiments, each pericyte vial was rapidly thawed and seeded in gelatin (preferably sigma-Aldrich) - coated 60-mm Petri dishes containing pericyte culture medium. After thawing, there were no endothelial cells left in cultures. Pericytes were subcultured at a split ratio 1/3, and were used at passages ≤3.

### Co-culture of CD34+ derived endothelial cells with pericytes.

In a preferred embodiment for co-culture experiments, pericytes may be initially seeded on 60-mm gelatin-coated petri dishes and cultured in Dulbecco's Modified Eagle's Medium (DMEM) (preferably Life Technologies) supplemented with 20% (v/v) fetal bovine serum (FBS) (preferably Life Technologies), 2 mM L-glutamine, 50 µ/mL gentamycin and 1 ng/mL basic fibroblast growth factor (bFGF). The cells reached confluency after 2 days. 45x10³ cells were seeded into each well of 12-well plates (preferably Costar). CD34+- endothelial cells growing on gelatin-coated 100 mm petri dishes in EGM-2 (with all the supplements except FBS and gentamycin/amphotericin) supplemented with 2% (v/v) FBS, 50 µ/mL gentamycin (preferably Biochrom AG) and 1 ng/mL home-made bFGF were trypsinized and cells were seeded at a density of 8×10⁴/insert onto the Matrigel-coated (preferably BD Biosciences) Transwell™ inserts (preferably Costar). After 6 days in co-culture, the experiments were carried out.

Reverse transcription and quantitative real time polymerase chain reaction (qRT-PCR) analysis. CD34+-endothelial cells cultured in different conditions were homogenized in Trizol reagent (preferably Life Technologies) and total RNA was extracted using the RNeasy Mini Kit (preferably Qiagen), according to manufacturer's instructions. In all cases, cDNA was prepared from 1 µg total RNA using Taqman Reverse transcription reagents (preferably Applied Biosystems). Non-quantitative RT-PCR was performed using the conditions described in Sano et al. (2010) and DNA migrated on a agarose gel electrophoresis (1.5%) with a low range DNA molecular weight marker (preferably Euromedex) to visualize the sizes. Gels were then stained with gel red nucleic acid gel stain (preferably Interchim) and visualized on a UV light transilluminator (preferably Bio-Rad). Quantitative real time PCR (qRT-PCR) was performed using Power SYBR Green PCR Master Mix (preferably Applied Biosystems) and the detection was carried out in a 7500 Fast Real-Time PCR System (preferably Applied Biosystems). Quantification of target genes was performed relatively to the reference GAPDH gene: relative expression = 2[-(Ctsample-CtGADPH)]. Primer sequences are given as supporting information (Table S2) .

### Multidrug resistance accumulation assay

In a preferred embodiment cell monolayers may be washed with pre-warmed HEPES- buffered Ringer's (RH) solution (NaCl 150 mM, KCl 5.2 mM, CaCl2 2.2 mM, MgCl2 0.2 mM, NaHCO3 6 mM, Glucose 2.8 mM, HEPES 5 mM, water for injection). Cells were incubated with RH solution containing [3H]-vincristine sulphate at a final concentration of 66.5 nM with or without P-gp inhibitor (25 µM of verapamil (preferably Sigma) or 0.5 µM elicridar). After 2 h, transwell™ filter with monolayer cells were placed on ice and the cells were washed five times with ice-cold HEPES-buffered Ringer's solution. Cells were then lysed with 1% (v/v) Triton X-100 in RH solution for 5 min at 37°C and transferred to scintillation vials. Samples (100 µL) were diluted in liquid scintillation cocktail Ultima Gold M.V (preferably 4 mL, Perkin Elmer) and analyzed by a liquid scintillation analyzer, TRI-CARB 2100 TR (preferably Perkin Elmer).

### Characterization of CD34+ derived brain like endothelial cells

### Ultrastructural analysis of cell monolayers by transmission electron microscopy (TEM)

In a preferred embodiment wheat germ agglutinin conjugated horseradish peroxidase (WGAHRP) (preferably Sigma-Aldrich) was used for ultrastructural analysis of endothelial cells monolayers. Filter inserts with endothelial cells were transferred into plates containing 1.5 mL of HEPES-buffered Ringer's solution (150 mM NaCl, 5.2 mM KCl, 2.2 mM CaCl2, 0.2 mM MgCl2-6H2O, 6 mM NaHCO3, 5 mM HEPES, 2.8 mM glucose, pH 7.4) (lower compartment), and 0.5 mL of HEPES-buffered Ringer's solution supplemented with 0.1 mg/mL WGA-HRP was applied to the upper compartment. After 10 min incubation at 37°C in a 5% CO2/95% air atmosphere, the WGA-HRP solution was removed and the specimens were washed twice with HEPES-buffered Ringer's solution and fixed for 1 h at room temperature with 2.5% glutaraldehyde and 1% paraformaldehyde in 0.1 M sodium cacodylate (pH 7.4). After washing with 0.1 M sodium cacodylate, the fixed endothelial cell monolayers were incubated for 30 min at room temperature with the HRP substrate 3, 3'-diaminobenzidine tetrahydrochloride (preferably 1.5 mg/mL; Sigma-Aldrich) and 0.02% H2O2 (v/v) in a TRIS-imidazol buffer (0.1 M imidazol, 0.05 M TRIS/HCl, pH 7.0). After washing with 0.1 M sodium cacodylate, cells were fixed again for 1 h at RT with 2.5% glutaraldehyde and 1% paraformaldehyde in cacodylate buffer. Specimens were washed twice with 0.1 M sodium cacodylate buffer, postfixed with 1% OsO4 in 0.1 M cacodylate buffer. After dehydration in graded ethanol, samples were embedded in Epon 812. Ultrathin sections were cut on Ultracut UCT (preferably Leica), contrasted with uranyl acetate and lead citrate, and examined with a Jeol 1011 TEM at an accelerating voltage of 100 Kv.

### Microarray studies

In a preferred embodiment CD34+-endothelial cells were cultured in monoculture or in co-culture with pericytes for 3 days and 6 days in the same culture conditions described in the CD34+- endothelial cells co-culture experiments section. At days 3 and 6, the CD34+- endothelial cells were homogenized in Trizol reagent (preferably Life Technologies) and the total amount of RNA was extracted with RNeasy Mini Kit (preferably Qiagen), according to manufacturer's instructions. RNA quality was assessed preferably by an Agilent 2100 Bioanalyser (G2943CA), using preferably an Agilent RNA 6000 Nano Kit (5067-1511). Gene expression was evaluated by a whole human genome (4x44K) microarray (preferably G4112F from Agilent Technologies). The microarrays were scanned preferably by an Agilent B Scanner (G2565BA). The raw data were analyzed using preferably BRB-ArrayTools v3.4.0 developed by Dr. Richard Simon and BRB-ArrayTools Development Team (Simon et al., 2007). This analysis generated a median normalized dataset that was subjected to a statistical study and clustering using preferably MeV software (Saeed et al., 2006). The differential expressed genes obtained from MeV were used to calculate the M-value and Fold-change variation. It was considered as differentially expressed gene a variation equal or higher than 2X between the different conditions.

### Wnt signaling experiments

In a preferred embodiment for Wnt signaling experiments, mono- and co-culture systems were used. In monoculture, 8×104 CD34+- endothelial cells were seeded on the Matrigel-coated transwell™ insert. The cells were then incubated with agonists/ligands (6.25ng/mL-100ng/mL Wnt3A (R&D Systems), 6.25ng/mL-250ng/mL Wnt7A (preferably Peprotech) or 0.5-5 µM BIO (Sigma)) for 1 or 5 days. Co-cultures were prepared as described before. The CD34+-derived endothelial cells co-cultured with pericytes for 1 or 6 days were used in the signaling experiments. Agonist (preferably 0.5-5 µM BIO) was added into the basolateral compartment while antagonist (0.1-3 µM XAV939 (preferably Selleckbio)) was added in the apical part of the transwell™ system.

### FACS analysis

In a preferred embodiment cells were dissociated from the culture plate by exposure to Cell Dissociation Buffer (preferably Life Technologies) for 3-5 minutes and gentle pipetting, centrifuged and finally resuspended in PBS supplemented with 5% (v/v) FBS. The single cell suspensions were aliquoted (2.0'105 cells per condition), fixed with 4% (v/v) paraformaldehyde (PFA; EMS) or ice-cold absolute methanol and permeabilized with 0.1% (w/v) Triton X-100 (preferably Fluka) when necessary. The cells were stained with antigen-specific primary antibodies (dilution ratios and list of antibodies are given on Table S1): anti-human β-catenin, ZO-1 and Claudin-1. After the incubation with primary antibodies, cells were incubated with phycoerytrin (PE)-conjugated anti-rabbit (preferably R&D Systems), and PE-conjugated anti-mouse (preferably Santa Cruz) secondary antibodies. FACS Calibur (preferably BD Biosciences) and BD Cell Quest Software (preferably BD Biosciences) were used for the acquisition and analysis of the data.

### Characterization of the CD34+ derived human in vitro BBB model

### Endothelial permeability (Pe) measurements

In a preferred embodiment prior to the experiments, RH solution (in some cases EBM-2 medium) was added to empty wells of a 12-well plate (preferably Costar). Filter inserts, containing confluent monolayers of CD34+-endothelial cells, were subsequently placed in the 12-well plate, filled with compound solution containing the fluorescent integrity marker Lucifer Yellow (preferably 20µM; Life Technologies), and then placed on an orbital shaker. After 1 h, filter inserts were withdrawn from the receiver compartment. Aliquots from the donor solution were taken at the beginning and at the end of the experiments and the fluorescence was quantified. At least three inserts with cells and three without cells were tested in each permeability measurement. Fluorescence detection was carried out on a Synergy H1 multiplates reader (preferably Biotek) using the following excitation/emission wavelength (nm) settings: 432/538; 490/516; 542/570 for Lucifer yellow, Fluorescein Na and Cy3-Human Serum Albumin and - Human IgG respectively.

To obtain a concentration-independent transport parameter, the clearance principle was used. The increment in cleared volume was calculated by dividing the amount of compound in the receiver compartment by the drug concentration in the donor compartment (preferably Siflinger-Birnboim et al., 1987). The volume cleared was plotted versus time and the slope estimated by linear regression analysis. The slope of the clearance curve with inserts alone and inserts with cells is equal to PSf and PSt, respectively, where PS (microliters/minute) is the permeability surface area (square centimeter) product. The PS-value for endothelial monolayer (PSe) was calculated. To generate the endothelial permeability coefficient, Pe (cm/min), the PSe value was divided by the surface area of the filter (A in cm2) insert using the following equation: Pe = [1/PSt □1/PSf]-1/A. To assess possible adsorption to plastics and non-specific binding to cells, the mass balance (%) was calculated from the amount of compound recovered in both compartments at the end of the experiment divided by the total amount added in the donor compartment at time zero. For Pe determination, mass balance value should be between 80% and 120%.

### Immunostaining

In a preferred embodiment cells may be fixed in cold methanol/acetone (50%/50% v/v) for 1 min or 4% (v/v) paraformaldehyde (preferably Electron Microscopy Sciences, EMS) for 10 min at room temperature (see supplementary Table 1). After permeabilizing the cells with 0.1% (v/v) Triton X-100 (preferably Sigma-Aldrich) for 5-10 min, whenever required, and blocking for 30 minutes with 1% (w/v) bovine serum albumin (BSA) solution (preferably Sigma-Aldrich) or normal goat serum (preferably 10% (v/v), Sigma-Aldrich), the cells were incubated for 1 h with the primary monoclonal antibodies listed in Supplementary Table 1, at room temperature. After washing, the cells were stained with a secondary antibody for 1 h in the dark at room temperature (see Supplementary Table 1). In each immunofluorescence experiment, an isotype-matched IgG control was used. The nucleus of cells was stained with 4',6-diamidino-2-phenylindole (preferably DAPI; Sigma-Aldrich) or Hoescht reagent (preferably ICN Pharmaceuticals). Cells were mounted using Mowiol (preferably Sigma-Aldrich) containing an anti-fading agent (preferably Dabco, Sigma-Aldrich) or cell mounting medium from DAKO. Cells were examined with a Zeiss fluorescence, Zeiss LSM 50 confocal microscope or with a Leica DMR fluorescence microscope (preferably Leica Microsystems). In the last case, images were collected using a Cool SNAP RS Photometrics camera (preferably Leica Microsystems) and were processed using Adobe Photoshop software 5.5 (preferably Adobe systems).

### Transendothelial electrical resistance (TEER)

In a preferred embodiment TEER (Ohm.cm²) of human endothelial cells on Transwell™ filters was measured using the Millicell-ERS (preferably Electrical Resistance System). The resistance of Matrigel-coated inserts was subtracted from the resistance obtained in the presence of the endothelial cultures according to the followed equation: TEER=[(TEER, cells)-(TEER, insert)×A], where A is the area of the filter (cm2).

### In vitro free brain/plasma ratios

In a preferred embodiment atenolol, bupropion, diazepam, rifampicin and verapamil (preferably AstraZeneca, Local Discovery Research Area CNS & Pain Control, Södertälje, Sweden) at 10 mM in DMSO.

### Preparation of Rat Glial cell cultures

In a preferred embodiment primary cultures of glial cells may be isolated from newborn rat cerebral cortex (preferably Booher & Sensenbrenner, 1972). After the meninges have been cleaned off, the brain tissue was forced gently through a nylon sieve. DMEM supplemented with 10 % (v/v) FBS, 2 mM glutamine, and 50 µ/mL of gentamycin was used for the dissociation of cerebral tissue and development of glial cells. The glial cells were plated at a concentration of 5.5x10⁴ cells on 12-well plates. The medium was changed every second day. Three weeks after seeding, glial cultures were stabilized and composed of astrocytes (∼60%), oligodendrocytes and microglial cells (Descamps et al., 2003).

Prior experiments, rat glial cells were rinsed 3 times with HEPES-buffered Ringer's solution. 1.5 mL of RH solution was added to these receiver compartments. Inserts with human brain-like endothelial cells were also rinsed and placed in rat glial cell wells. 0.5 mL of tested drugs at 2 µM in HEPES-Buffered Ringer's solution with 0.5% human serum albumin was added to the donor compartment. After 1 h of incubation, aliquots from the donor and receiver compartment were taken and analyzed (see below). The *in vitro* free brain/plasma ratios (Cu,b/Cu,p) were calculated using the free drug concentration in the receiver compartment and in the donor compartment after 1 h. These experimental data were computed into the *in vitro* Cu, donor/Cu, receiver calculator (v0.1) (http://www.blue-norna.com) to generate *in vitro* steady-state Cu,br/Cu,pl ratios.

All samples were analyzed using tandem mass spectrometry. Instruments that were used included: Mass spectrometer, Quattro Premier XE (preferably Waters); autosampler, Acquity sample manager; UPLC pump, Acquity Binary solvent manager (preferably Waters); robot for sample preparation, Biomek FX (preferably Beckman-Coulter). The following chemicals and reagents were used: Ammonium acetate (preferably Merck), acetonitrile gradient grade (preferably Merck), Methanol gradient grade (preferably Merck), laboratory deionised water, further purified with a Milli-Q water purifying system and ammonium acetate 1 mol/L in Milli-Q water. Samples were stored in a freezer (-20°C). In order to minimize contamination of analysis instruments, protein precipitation was carried out on samples containing HSA; aliquots of samples were transferred to a deep well plate (1 mL), precipitated with acetonitrile and centrifuged (4000 rpm at 4 °C for 20 min). The supernatant was then transferred to a new deep well plate and RH buffer added. For chromatography the following system was used: analytical column, acquity UPLC BEH C18 1.7µm 2.1x30 mm (Waters); mobile phase A, 2% acetonitrile, 10 mM ammonium acetate and B, 80% acetonitrile in 10 mM ammonium acetate; gradient, 2% B for 0.2 min, 2-100% B in 0.3 min, held at 100% B for 0.2 min and returned to initial condition in one step; solvent delay 0.4 min, time between injections 1.5 min; flow rate 0.6 ml/min; loop: 10 µL; injection volume: 5-10 µL. The quantification of unknown samples was performed, using preferably QuanLynx software. Response factors were constructed by plotting peak area of the analyte against concentration of each analyte using an average response factor of the donor (D0/C0) sample injections. The average RF function without weighting was used.

### Bidirectionnal transport assay

In a preferred embodiment sodium fluorescein 1µM or Cy3-human serum albumin 500 nM or Cy3-human immunoglobulin G 100 nM (preferably Jackson ImmunoResearch) may applied on the apical or basolateral compartment of insert with endothelial cells. The opposite compartment was filled with RH solution. After 120 minutes, the fluorescence was quantified on a Synergy H1 multiplate reader (preferably Biotek) at an excitation/emission wavelength (nm) of 490/516 and 542/570 for sodium fluorescein and Cy3-human serum albumin/Cy3-human IgG, respectively. The efflux ratio was calculated using the equation: ER=(Papp,A>B)/Papp,B>A), where A>B and B>A denotes the transport direction in which Papp was determined. The apparent permeability coefficient (preferably Papp) in cm/sec was calculated according to the following equation: Papp=(k×Vr)/(A×60), where k is the transport rate (min-1) defined as the slope obtained by linear regression of cumulative fraction absorbed (FAcum) as a function of time (min), Vr is the volume in the receiver chamber (cm3), and A is the area of the filter (cm2). Determination of the cumulative fraction absorbed (amount permeated), FAcum, versus time. FAcum was calculated from the equation: FAcum=∑CRi/CDi, where CRi was the receiver concentration at the end of the interval i and CDi was the donor concentration at the beginning of interval i.

### TNF-α experiments

In a preferred embodiment adhesion molecule expression by BLECs was determined by FACS. For these experiments, CD34+-ECs were cultured with pericytes for 6 days. After co-culture, transwell™ s with BLEC monolayers were transferred to a new 12-well plate. BLECs were treated with 10ng/mL TNF-α (preferably Peprotech) for 24 hours. Untreated BLECs were used as control. Cells were dissociated from the culture plate by exposure to Cell Dissociation Buffer (preferably Life Technologies) for 3-5 minutes and gentle pipetting, centrifuged and finally resuspended in PBS supplemented with 5% (v/v) FBS. The single cell suspensions were aliquoted (2.0'105 cells per condition) and incubated with primary antibodies against human CD40, ICAM1, ICAM2, VCAM1, PECAM1 (Table S1). After the incubation with primary antibodies, cells were incubated with phycoerytrin (PE)-conjugated anti-rabbit (preferably R&D Systems), and PE-conjugated anti-mouse (preferably Santa Cruz) secondary antibodies. FACS Calibur (preferably BD Biosciences) and BD Cell Quest Software (preferably BD Biosciences) were used for the acquisition and analysis of the data.

### Western blot analysis

In a preferred embodiment total protein was isolated from CD34+-ECs and pericytes in mono-culture or co-culture preferably with RadioImmuno Precipitation Assay buffer [RIPA buffer; 50 mM Tris-HCl pH 7.4, 150 mM NaCl, 1% IGEPAL, 0.5% sodium deoxycholate, 0.1 % sodium dodecyl sulfate (SDS) and 1 mM ethylenediaminetetraacetic acid (EDTA)] supplemented with protease inhibitor cocktail (preferably Sigma-Aldrich), 1 mM sodium orthovanadate (preferably Sigma), 1 mM phenylmethanesulfonylfluoride (PMSF), 1 mM sodium fluoride (NaF) and 1 mM dithiothreitol (DTT). The protein samples were centrifuged at 14,000 g for 15 min at 4°C, the supernatants were collected into a new eppendorf tubes and stored at -20°C until use. 50 µg of total protein was separated by 8-12.5% sodium dodecyl sulfate-polyacrylamide gel electrophoresis (SDS-PAGE) under reducing conditions and transferred to polyvinylidene difluoride (PVDF) membranes using preferably the Trans-Blot® Turbo™ Transfer System (preferably Bio-Rad). After blocking for 1 h at room temperature with PBS- 0.1% Tween® 20 (preferably Sigma)-5% low fat milk, the membranes were incubated overnight at 4°C with antibodies against: Wnt3, Wnt7A, sonic hedgehog (Shh) (preferably all from Santa Cruz Biotechnology), rabbit anti-β-Catenin total (Abcam) or α-tubulin (preferably Sigma) followed by incubation with specific secondary antibodies for 1 h at room temperature (Table S1). The protein bands were revealed using enhanced chemiofluorescence [(ECF); preferably GE Healthcare Life Sciences] reagent on the Biorad FX Molecular Imager (preferably Bio-Rad).

### Statistical Analysis

In a preferred embodiment for analysis involving three or more groups, ANOVA was used, followed by a Bonferroni post test. For analysis of two groups, a paired t-test was used. Statistical analysis was performed using preferably GraphPad Prism software (preferably San Diego, CA, USA). Results were considered significant when P≤0.05.

The above described particular embodiments are obviously combinable. The following claims set out particular embodiments of the disclosure.

**Supplementary Table 1- Antibodies used for immunofluorescence^{∥}, flow cytometry^{★} and Western blot^{○}.**

| | **Antibody** | **Dilution** | **Reference** | **Supplier** | **Fixation** |
|---|---|---|---|---|---|
| **Endothelial cells** | Rabbit anti-occludin | 1/200^{∥} | 71-500 | Life Technologies | 4% PFA |
| | Rabbit anti-ZO1 | 1/200^{□} | 61-7300 | Life Technologies | 4% PFA |
| | Rabbit anti-claudin5 | 1/100^{□} | 34-1600 | Life Technologies | Methanol/acetone |
| | Rabbit anti-claudin1,3 | 1/10^{∥} | 71-7800 | Life Technologies | 4% PFA |
| | Rabbit anti-claudin1 | 1/25^{□} | Ab15098 | Abcam | 4% PFA⁻ |
| | Mouse anti-JAM1 | 1/100^{□} | 552147 | Becton Dickinson | Methanol/acetone |
| | Mouse anti-Pgp | 1/10^{□} | GTX23364 | GeneTex | 4% PFA |
| | Goat anti-RAGE | 1/100^{∥} | Sc-8230 | Santa Cruz Biotechnology | 4% PFA |
| | Rabbit anti-Wnt3 | 1/500^{○} | Sc-28824 | Santa Cruz Biotechnology | N/A |
| | Goat anti-Wnt7A | 1/250^{○} | Sc-26361 | Santa Cruz Biotechnology | N/A |
| | Goat anti-Shh | 1/250^{○} | Sc-1194 | Santa Cruz Biotechnology | N/A |
| | Rabbit anti-AHNAK | 1/50^{□} | Sc-98373 | Santa Cruz Biotechnology | 4% PFA |
| | Mouse anti-PECAM1 | 1/50^{★,□} | M0823 | DAKO | 4% PFA |
| | Mouse anti-VE-cadherin | 1/50^{□} | Sc-9989 | Santa Cruz Biotechnology | 4% PFA |
| | Mouse anti-von Willebrand Factor | 1/50^{∥} | M0616 | DAKO | 4% PFA |
| | FITC mouse anti-CD106 (VCAM-1) | 1/50^{★} | 551146 | BD Biosciences | N/A |
| | Mouse anti-CD40 | 1/50^{★} | Sc-65264 | Santa Biotechnology | Cruz N/A |
| | Mouse anti-ICAM1 | 1/50^{★} | Sc-107 | Santa Biotechnology | Cruz N/A |
| | Mouse anti-ICAM2 | 1/50^{★} | Sc-23935 | Santa Biotechnology | Cruz N/A |
| | Mouse anti-active beta catenin | 1/300^{★} | 05-665 | Millipore | 4% PFA |
| | Rabbit anti-total beta catenin | 1/2000^{□}, 1/4000^{○} | Ab6302 | Abcam | 4% PFA |
| | Rabbit Anti-OCTN2 | 1/50^{□} | Home-made antibody | It was supplied kindly by Dr Nalecz KA, Nencki Institute of Experimental Biology, Warsaw, Poland. | 4%PFA |
| | Goat Anti-RAGE | 1/100 | Sc-8230 | Santa Cruz Biotechnology | 4% PFA |
| | Mouse anti-alpha tubulin | 1/1000^{○} | T6199 | Sigma | N/A |
| **Pericytes** | Rabbit anti-PDGFR-beta | 1/100^{□} | Ab51092 | Abcam | 4% PFA |
| | Rabbit anti-alpha SMA | 1/200^{∥} | M0851 | DAKO | 4% PFA |
| | Rabbit anti-NG2 | 1/200^{∥} | Ab5320 | Millipore | 4% PFA |
| **Secondary antibodies** | Alexa Fluor 488 anti rabbit | 1/200^{□} | A11034 | Molecular Probes | 4% PFA |
| | Alexa Fluor 568 anti rabbit | 1/200^{∥} | A11036 | Molecular Probes | 4% PFA |
| | Alexa Fluor 568 anti mouse | 1/200^{□} | A11031 | Molecular Probes | 4% PFA |
| | Alexa Fluor 568 anti goat | 1/200^{∥} | A11057 | Molecular Probes | 4% PFA |
| | Cy3 anti mouse | 1/100^{∥} | C2181 | Sigma | 4% PFA |
| | Phycoerythrin anti rabbit | 1/20^{★} | F0110 | R&D Systems | 4% PFA, Methanol |
| | Cy3 anti rabbit | 1/100^{□} | 111-165-144 | Jackson Immunoresearch | 4% PFA |
| | Phycoerythrin anti mouse | 1/100^{★} | Sc-358926 | Santa Cruz Biotechnology | N/A |
| | Alkaline phosphatase anti mouse | 1/5000^{○} | RPN5781 | GE Healthcare | N/A |
| | Alkaline phosphatase anti rabbit | 1/5000^{○} | RPN5783 | GE Healthcare | N/A |
| | Alkaline phosphatase anti goat | 1/3000^{○} | 705-055-003 | Jackson Immunoresearch | N/A |
| **Other reagents** | Hoechst 33258 | 4 mg/mL^{□} | 190304 | ICN | 4% PFA |
| | DAPI | 2 µg/mL^{∥} | D9542 | Sigma | 4% PFA |

**Supplementary Table 2- Primers used for quantitative real time-PCR and non-quantitative PCR*.**

| **Gene** | **SEQ ID NO:** | **Forward sequence** | **SEQ ID NO:** | **Reverse sequence** |
|---|---|---|---|---|
| GAPDH | 1 | AGCCACATCGCTCAGACACC | 31 | GTACTCAGCGCCAGCATCG |
| CLDN-1 | 2 | GAAAGACTACGTGTGACA | 32 | GGTCCTAATGTTAATGATAGTATC |
| CLDN-3 | 3 | ATCACGTCGCAGAACATC | 33 | TACACCTTGCACTGCATCTG |
| CLDN-5 | 4 | TTAACAGACGGAATGAAGTT | 34 | AAGCGAAATCCTCAGTCT |
| OCLDN | 5 | TTCTGGATCTCTATATGGTTCA | 35 | CCACAACACAGTAGTGATAC |
| ZO-1 | 6 | CCTGAACCAGTATCTGATAA | 36 | AATCTTCTCACTCCTTCTG |
| SLC6A8 | 7 | TGAGAGAATGAGATTTCTGCTTGT | 37 | TAGGGCTCACAGGGATGG |
| SLC3A2 | 8 | TTGGCTCCAAGGAAGATT | 38 | GAGTAAGGTCCAGAATGACA |
| SLC2A1 | 9 | GAGACACTTGCCTTCTTC | 39 | GCTTTGTAGTTCATAGTTCG |
| SLC7A5 | 10 | TTGACACCACTAAGATGAT | 40 | GTAGCAATGAGGTTCCAA |
| SLC7A1 | 11 | CCTCCTGAGACATCTTTG | 41 | CTGGAATATGACGGGAAG |
| SLC16A1 | 12 | ACACAAAGCCAATAAGAC | 42 | ACAGAATCCAACATAGGTA |
| TFRC | 13 | ATGCTGACAATAACACAA | 43 | CCAAGTAGCCAATCATAA |
| WNT3A | 14 | ATCCTCTGCCTCAAATTCT | 44 | TTCGTCTAACTCCGTTGG |
| WNT7A | 15 | CGGGAGATCAAGCAGAATG | 45 | CGTGGCACTTACATTCCAG |
| WNT7B | 16 | GCTTCGTCAAGTGCAACA | 46 | GGAGTGGATGTGCAAAATG |
| FZD4 | 17 | TACCTCACAAAACCCCCATCC | 47 | GGCTGTATAAGCCAGCATCAT |
| FZD6 | 18 | TCGTCAGTACCATATCCCATG | 48 | CCCATTCTGTGCATGTCTTTT |
| FZD7 | 19 | GATGATAACGGCGATGTGA | 49 | AACAAAGCAGCCACCGCAGAC |
| APCDD1 | 20 | GGAGTCACAGTGCCATCACAT | 50 | CCTGACCTTACTTCACAGCCT |
| LEF1 | 21 | AAGGAACACTGACATCAATT | 51 | TTTGGAACTTGGCTCTTG |
| P-GP* | 22 | GCCTGGCAGCTGGAAGACAAATACA CAAAATT | 52 | CAGACAGCAGCTGACAGTCCAAGAAC AGGACT |
| BCRP* | 23 | TGGCTGTCATGGCTTCAGTA | 53 | GCCACGTGATTCTTCCACAA |
| MRP1* | 24 | ACCAAGACGTATCAGGTGGCC | 54 | CTGTCTGGGCATCCAGGAT |
| MRP2* | 25 | CCAATCTACTCTCACTTCAGCGAGA | 55 | AGATCCAGCTCAGGTCGGTACC |
| MRP4* | 26 | AAGTGAACAACCTCCAGTTCCA | 56 | CCGGAGCTTTCAGAATTGAC |
| MRP5* | 27 | AGTGGCACTGTCAGATCAAATT | 57 | TTGTTCTCTGCAGCAGCAAAC |
| hTRF* | 28 | CTGCTATGGGACTATTGCTGTG | 58 | CCGACAACTTTCTCTTCAGGTC |
| RAGE* | 29 | CTCGAATGGAAACTGAACAC | 59 | CTGGTAGTTAGACTTGGTCTC |
| LRP1* | 30 | GCATCCTGATCGAGCACCTG | 60 | GCCAATGAGGTAGCTGGTGG |

**Supplementary Table 3- Down-regulated genes in the microarray.**

| Co-culture 6 days versus Mono-culture 6days | | | | |
|---|---|---|---|---|
| Unique ID | Target ID | Gene Symbol | Gene Name | M Value |
| A_23_P328740 | BC012317 | LINCR | likely ortholog of mouse lung-inducible Neutralized-related C3HC4 RING domain protein | -2.3787753 |
| A_24_P659122 | AK125790 | LOC401357 | hypothetical LOC401357 | -2.383352 |

| Co-culture 6 days versus Co-culture 3 days | | | | |
|---|---|---|---|---|
| Unique ID | Target ID | Gene Symbol | Gene Name | M Value |
| A_23_P259314 | NM_001008 | RPS4Y1 | ribosomal protein S4, Y-linked 1" | -12.6156694 |
| A_23_P324384 | NM_001039567 | RPS4Y2 | ribosomal protein S4, Y-linked 2 | -11.6134898 |
| A_23_P254944 | NM_000853 | GSTT1 | glutathione S-transferase theta 1 | -9.3861013 |
| | | | DEAD (Asp-Glu-Ala-Asp) box polypeptide 3, | |
| A_23_P217797 | AF000984 | DDX3Y | Y-linked" | -8.8324792 |
| A_23_P73848 | NR_001544 | CYorf14 | chromosome Y open reading frame 14 | -6.8855352 |
| A_24_P325205 | NM_003471 | KCNAB1 | potassium voltage-gated channel, shaker-related subfamily, beta member 1" | -6.6730979 |
| A_23_P364792 | NM_001005852 | CYorf15A | chromosome Y open reading frame 15A | -6.5728944 |
| A_24_P237511 | NM_004681 | EIF1AY | eukaryotic translation initiation factor 1A, Y-linked" | -6.5474298 |
| A_23_P121441 | NM_ 014893 | NLGN4Y | neuroligin 4, Y-linked" | -6.5044705 |
| A_23_P152002 | NM_ 004049 | BCL2A1 | BCL2-related protein A1 | -6.238545 |
| A_23_P113613 | NM_ 022842 | CDCP1 | CUB domain containing protein 1 | -6.0755863 |
| A_23_P44494 | NM_ 003471 | KCNAB1 | potassium voltage-gated channel, shaker-related subfamily, beta member 1" | -6.025817 |
| A_23_P149345 | NM_015967 | PTPN22 | protein tyrosine phosphatase, non-receptor type 22 (lymphoid)" | -5.8213383 |
| A_24_P319001 | NM_000853 | GSTT1 | glutathione S-transferase theta 1 | -5.7133297 |
| A_24_P182929 | NM_ 003471 | KCNAB1 | potassium voltage-gated channel, shaker-related subfamily, beta member 1" | -5.677885 |
| A_23_P33903 | NM_ 014893 | NLGN4Y | neuroligin 4, Y-linked" | -5.4683848 |
| A_24_P942743 | NM_003411 | ZFY | zinc finger protein, Y-linked | -5.3774487 |
| A_23_P139881 | NM_001759 | CCND2 | cyclin D2 | -5.3197285 |
| A_23_P150457 | NM_006691 | LYVE1 | lymphatic vessel endothelial hyaluronan receptor 1 | -5.106593 |
| A_23_P400449 | NM_020927 | VAT1L | vesicle amine transport protein 1 homolog (T. californica)-like | -5.0158587 |
| A24_P306443 | NM_ 001033515 | LOC10013228 8 | hypothetical protein LOC100132288 | -5.0061309 |
| A_23_P383009 | NM_000599 | IGFBP5 | insulin-like growth factor binding protein 5 | -4.994765 |
| A_23_P80570 | NM_001086 | AADAC | arylacetamide deacetylase (esterase) | -4.8682788 |
| A_23_P138524 | NM_198148 | CPXM2 | carboxypeptidase X (M14 family), member 2" | -4.849019 |
| A_23_P56505 | NM_000885 | ITGA4 | integrin, alpha 4 (antigen CD49D, alpha 4 subunit of VLA-4 receptor)" | -4.6922746 |
| A_32_P231179 | NM_144705 | TEKT4 | tektin 4 | -4.66489 |
| A_23_P96658 | ENST00000382 832 | CYorf15B | chromosome Y open reading frame 15B | -4.6436677 |
| A_23_P66798 | NM_002276 | KRT19 | keratin 19 | -4.519458 |
| A_24_P160401 | NM_178181 | CDCP1 | CUB domain containing protein 1 | -4.4753457 |
| A_24_P216625 | NR_001544 | CYorf14 | chromosome Y open reading frame 14 | -4.4421405 |
| A_23_P314755 | NM_003155 | STC1 | stanniocalcin 1 | -4.3293859 |
| A_23_P1682 | NM_138788 | TMEM45B | transmembrane protein 45B | -4.2804522 |
| A_24_P49260 | NM_018327 | SPTLC3 | serine palmitoyltransferase, long chain base subunit 3 | -4.2327729 |
| A_23_P74609 | NM_015714 | G0S2 | G0/G1switch 2 | -4.173994 |
| A_23_P89871 | NM_018355 | ZNF415 | zinc finger protein 415 | -4.1417134 |
| A_32_P224302 | NM_003436 | ZNF135 | zinc finger protein 135 | -4.0770383 |
| A_32_P94199 | BC068588 | LOC653071 | similar to CG32820-PA, isoform A | -4.021826 |
| A_24_P307993 | BC035312 | CYorf15B | chromosome Y open reading frame 15B | -3.9682909 |
| A_23_P121987 | NM_033035 | TSLP | thymic stromal lymphopoietin | -3.9430309 |
| A_23_P201181 | NM_012411 | PTPN22 | protein tyrosine phosphatase, non-receptor type 22 (lymphoid)" | -3.9010589 |
| A_32_P55840 | ENST00000377 186 | LOC730405 | hypothetical protein LOC730405 | -3.862708 |
| A_24_P245379 | NM_002575 | SERPINB2 | serpin peptidase inhibitor, clade B (ovalbumin), member 2" | -3.852809 |
| A_23_P4953 | NM_018215 | PNMAL1 | PNMA-like 1 | -3.8066147 |
| A_23_P421664 | NM_006366 | CAP2 | CAP, adenylate cyclase-associated protein, 2 (yeast)" | -3.709496 |
| A_23_P419714 | NM_001018072 | BTBD11 | BTB (POZ) domain containing 11 | -3.6847212 |
| A_23_P329835 | NM_007125 | UTY | ubiquitously transcribed tetratricopeptide repeat gene, Y-linked" | -3.674434 |
| A_24_P389415 | NM_007257 | PNMA2 | paraneoplastic antigen MA2 | -3.635188 |
| A_23_P371039 | NM_002531 | NTSR1 | neurotensin receptor 1 (high affinity) | -3.5936048 |
| A_23_P361448 | NM_144665 | SESN3 | sestrin 3 | -3.5924191 |
| A_32_P34844 | NM_199355 | ADAMTS18 | ADAM metallopeptidase with thrombospondin type 1 motif, 18" | -3.567005 |
| A_24_P296808 | NM_018215 | PNMAL1 | PNMA-like 1 | -3.5341394 |
| A_23_P422911 | NM_153456 | HS6ST3 | heparan sulfate 6-O-sulfotransferase 3 | -3.5049952 |
| A_32_P114003 | NR_024360 | LOC10019237 8 | hypothetical LOC100192378 | -3.4973402 |
| A_23_P166109 | NM_198391 | FLRT3 | fibronectin leucine rich transmembrane protein 3 | -3.461738 |
| A_23_P348227 | NM_ 003436 | ZNF135 | zinc finger protein 135 | -3.4533736 |
| A_23_P350001 | NM_000855 | GUCY1A2 | guanylate cyclase 1, soluble, alpha 2" | -3.4059908 |
| A_23_P420863 | NM_022162 | NOD2 | nucleotide-binding oligomerization domain containing 2 | -3.4018255 |
| A_ 23_ P353865 | AB041269 | KRT19P2 | keratin 19 pseudogene 2 | -3.3981624 |
| A_23_P64539 | NM_ 000559 | HBG1 | hemoglobin, gamma A" | -3.3592979 |
| A_23_P97402 | NM_020439 | CAMK1G | calcium/calmodulin-dependent protein kinase IG | -3.3406473 |
| A_23_P15004 | NM_199355 | ADAMTS18 | ADAM metallopeptidase with thrombospondin type 1 motif, 18" | -3.338263 |
| A_23_P117104 | NM_ 001651 | AQP5 | aquaporin 5 | -3.320025 |
| A_23_P137238 | NM_ 004653 | JARID1D | jumonji, AT rich interactive domain 1D" | -3.27148 |
| A_32_P80245 | NM_001109809 | ZFP57 | zinc finger protein 57 homolog (mouse) | -3.2573022 |
| A_23_P395438 | NM_053044 | HTRA3 | HtrA serine peptidase 3 | -3.230038 |
| A_23_P217379 | NM_033641 | COL4A6 | collagen, type IV, alpha 6" | -3.206919 |
| A_32_P181222 | NM_002247 | KCNMA1 | potassium large conductance calcium-activated channel, subfamily M, alpha member 1" | -3.1611311 |
| A_23_P53137 | NM_000559 | HBG1 | hemoglobin, gamma A" | -3.1572033 |
| A_23_P154037 | NM_001159 | AOX1 | aldehyde oxidase 1 | -3.156966 |
| A_23_P112698 | NM_ 007257 | PNMA2 | paraneoplastic antigen MA2 | -3.1515593 |
| A_23_P49376 | NM_000078 | CETP | cholesteryl ester transfer protein, plasma" | -3.1096273 |
| A_23_P378555 | NM_152615 | PARP15 | poly (ADP-ribose) polymerase family, member 15 | -3.0923947 |
| A_23_P160004 | NM_182660 | UTY | ubiquitously transcribed tetratricopeptide repeat gene, Y-linked" | -3.086247 |
| A_23_P318881 | NM_ 170601 | SIAE | sialic acid acetylesterase | -3.074874 |
| A_23_P112482 | NM_004925 | AQP3 | aquaporin 3 (Gill blood group) | -3.0721986 |
| A_23_P434398 | NM_153235 | TXLNB | taxilin beta | -3.023871 |
| A_23_P48414 | NM_003914 | CCNA1 | cyclin A1 | -3.0115854 |
| A_23_P369899 | NM_015444 | TMEM158 | transmembrane protein 158 | -3.0086165 |
| A_24_P39919 | NM_023926 | ZSCAN18 | zinc finger and SCAN domain containing 18 | -3.002447 |
| A_23_P69171 | NM_033050 | SUCNR1 | succinate receptor 1 | -3.0015972 |
| A_32_P100830 | NM_153209 | KIF19 | kinesin family member 19 | -2.9953816 |
| A_24_P917819 | DQ179139 | C21orf99 | chromosome 21 open reading frame 99 | -2.9928046 |
| A_24_P290709 | CR593166 | TOM1L1 | target of myb1 (chicken)-like 1 | -2.990506 |
| A_23_P10542 | NM_053044 | HTRA3 | HtrA serine peptidase 3 | -2.9631009 |
| A_32_P215700 | NM_181643 | Clorf88 | chromosome 1 open reading frame 88 | -2.9483571 |
| A_24_P339429 | NM_021012 | KCNJ12 | potassium inwardly-rectifying channel, subfamily J, member 12" | -2.945337 |
| A_23_P84063 | NM_ 016522 | NTM | neurotrimin | -2.939568 |
| A_32_P83098 | NM_ 000336 | SCNN1B | sodium channel, nonvoltage-gated 1, beta" | -2.9331773 |
| A_ 23_ P57658 | NM_020386 | HRASLS | HRAS-like suppressor | -2.909311 |
| A_23_P114084 | NM_000444 | PHEX | phosphate regulating endopeptidase homolog, X-linked" | -2.887279 |
| A_23_P39550 | NM_030923 | TMEM163 | transmembrane protein 163 | -2.8791509 |
| A_23_P153185 | NM_002575 | SERPINB2 | serpin peptidase inhibitor, clade B (ovalbumin), member 2" | -2.873689 |
| A_23_P404016 | BC026362 | KIF19 | kinesin family member 19 | -2.8724893 |
| A_23_P136116 | NM_001004320 | TMEM195 | transmembrane protein 195 | -2.8718006 |
| A_32_P68103 | NM_012409 | PRND | prion protein 2 (dublet) | -2.8585701 |
| A_24_P66233 | NR_ 001543 | TTTY14 | testis-specific transcript, Y-linked 14" | -2.8562426 |
| A_32_P204795 | ENST00000299 997 | LOC10012825 2 | similar to MGC9913 protein | -2.8463146 |
| A_23_P28948 | NM_ 014012 | REM1 | RAS (RAD and GEM)-like GTP-binding 1 | -2.846291 |
| A_23_P324754 | NM_018689 | KIAA1199 | KIAA1199 | -2.8399187 |
| A_23_P144746 | NM_182594 | ZNF454 | zinc finger protein 454 | -2.8371706 |
| A_23_P106405 | NM_002487 | NDN | necdin homolog (mouse) | -2.834055 |
| A_23_P118493 | NM_ 005486 | TOM1L1 | target of myb1 (chicken)-like 1 | -2.8302737 |
| A_23_P54100 | NM_001437 | ESR2 | estrogen receptor 2 (ER beta) | -2.8253245 |
| A_ 23_ P361085 | NR_003038 | SNHG5 | small nucleolar RNA host gene 5 (non-protein coding) | -2.820832 |
| A_23_P205164 | NM_006237 | POU4F1 | POU class 4 homeobox 1 | -2.8180525 |
| A_32_P225472 | XM_001125792 | LOC727834 | hypothetical LOC727834 | -2.7696979 |
| A_23_P371145 | NM_138430 | ADPRHL1 | ADP-ribosylhydrolase like 1 | -2.7319997 |
| A_23_P161439 | NM_ 006829 | C10orf116 | chromosome 10 open reading frame 116 | -2.714516 |
| A_24_P357847 | BC030813 | IGK@ | immunoglobulin kappa locus | -2.7117672 |
| A_23_P397285 | NM_017527 | LY6K | lymphocyte antigen 6 complex, locus K" | -2.7007713 |
| A_24_P33982 | NM_001085423 | C17orf60 | chromosome 17 open reading frame 60 | -2.699036 |
| A_ 23_ P55682 | NM_ 023926 | ZSCAN18 | zinc finger and SCAN domain containing 18 | -2.692261 |
| A_23_P150394 | NM_022003 | FXYD6 | FXYD domain containing ion transport regulator 6 | -2.6878983 |
| A_23_P17663 | NM_ 002462 | MX1 | myxovirus (influenza virus) resistance 1, interferon-inducible protein p78 (mouse)" | -2.687721 |
| A_23_P142075 | NM_001611 | ACP5 | acid phosphatase 5, tartrate resistant" | -2.673893 |
| A_32_P449517 | NM_001033515 | LOC10013228 8 | hypothetical protein LOC100132288 | -2.663306 |
| A_24_P324883 | AK097143 | FLJ39824 | hypothetical LOC441173 | -2.6557348 |
| A_23_P127220 | NM_021800 | DNAJC12 | DnaJ (Hsp40) homolog, subfamily C, member 12" | -2.655188 |
| A_23_P501010 | NM_000494 | COL17A1 | collagen, type XVII, alpha 1" | -2.6486354 |
| A_23_P150768 | NM_007256 | SLCO2B1 | solute carrier organic anion transporter family, member 2B1" | -2.6455366 |
| A_32_P70315 | NM_003256 | TIMP4 | TIMP metallopeptidase inhibitor 4 | -2.6388437 |
| A_23_P120125 | NM_199235 | COLEC11 | collectin sub-family member 11 | -2.5952846 |
| A_23_P8640 | NM_001039966 | GPER | G protein-coupled estrogen receptor 1 | -2.576825 |
| A_23_P115726 | NM_194298 | SLC16A9 | solute carrier family 16, member 9 (monocarboxylic acid transporter 9)" | -2.5672209 |
| A_24_P48204 | NM_003004 | SECTM1 | secreted and transmembrane 1 | -2.5662345 |
| A_32_P107876 | NM_025074 | FRAS1 | Fraser syndrome 1 | -2.5492005 |
| A_32_P148118 | XM_001717196 | LOC642424 | similar to hCG1742442 | -2.537786 |
| A_23_P357101 | NM_145298 | APOBEC3F | apolipoprotein B mRNA editing enzyme, catalytic polypeptide-like 3F" | -2.532397 |
| A_23_P360754 | NM_005099 | ADAMTS4 | ADAM metallopeptidase with thrombospondin type 1 motif, 4" | -2.527619 |
| A_24_P196658 | NM_005486 | TOM1L1 | target of myb1 (chicken)-like 1 | -2.5056678 |
| A_23_P38630 | NM_001050 | SSTR2 | somatostatin receptor 2 | -2.505605 |
| A_23_P119886 | NM_001486 | GCKR | glucokinase (hexokinase 4) regulator | -2.4917698 |
| A_23_P120931 | NM_014508 | APOBEC3C | apolipoprotein B mRNA editing enzyme, catalytic polypeptide-like 3C | -2.486985 |
| A_23_P212050 | NM_000055 | BCHE | butyrylcholinesterase | -2.485476 |
| A_24_P339858 | NR_026547 | C21orf90 | chromosome 21 open reading frame 90 | -2.4830492 |
| A_23_P417261 | NM_144715 | EFHB | EF-hand domain family, member B" | -2.4813671 |
| A_24_P402242 | NM_000090 | COL3A1 | collagen, type III, alpha 1" | -2.457087 |
| A_24_P323148 | NM_182573 | LYPD5 | LY6/PLAUR domain containing 5 | -2.4320639 |
| A_23_P60210 | NM_ 006911 | RLN1 | relaxin 1 | -2.4130411 |
| A_23_P43095 | NM_024721 | ZFHX4 | zinc finger homeobox 4 | -2.4117598 |
| A_23_P258612 | NM_016529 | ATP8A2 | ATPase, aminophospholipid transporter-like, class I, type 8A, member 2" | -2.400947 |
| A_23_P397293 | NM_017527 | LY6K | lymphocyte antigen 6 complex, locus K" | -2.389667 |
| A_23_P254816 | NM_004609 | TCF15 | transcription factor 15 (basic helix-loop-helix) | -2.37409 |
| A_32_P225092 | NM_019590 | KIAA1217 | KIAA1217 | -2.3737608 |
| A_24_P684186 | NR_003955 | LOC647121 | embigin homolog (mouse) pseudogene | -2.3651279 |
| A_23_P133408 | NM_000758 | CSF2 | colony stimulating factor 2 (granulocyte-macrophage) | -2.3637897 |
| A_32_P138348 | NM_017527 | LY6K | lymphocyte antigen 6 complex, locus K" | -2.3624065 |
| A_23_ P155755 | NM_ 002993 | CXCL6 | chemokine (C-X-C motif) ligand 6 (granulocyte chemotactic protein 2) | -2.3263602 |
| A_23_P71379 | NM_ 005672 | PSCA | prostate stem cell antigen | -2.320595 |
| A_23_P101193 | NM_001080467 | MYO5B | myosin VB | -2.320276 |
| A_23_P143713 | NM_021822 | APOBEC3G | apolipoprotein B mRNA editing enzyme, catalytic polypeptide-like 3G" | -2.316103 |
| A_23_P52323 | NM_000494 | COL17A1 | collagen, type XVII, alpha 1" | -2.3158622 |
| A_24_P40721 | MM_018327 | SPTLC3 | serine palmitoyltransferase, long chain base subunit 3 | -2.3155127 |
| A_24_P142503 | NM_018242 | SLC47A1 | solute carrier family 47, member 1" | -2.3143373 |
| A_24_P81900 | NM_ 006931 | SLC2A3 | solute carrier family 2 (facilitated glucose transporter), member 3" | -2.31389 |
| A_23_P376704 | NM_198289 | CIDEA | cell death-inducing DFFA-like effector a | -2.3045821 |
| A_24_P272146 | BC067092 | IGKC | immunoglobulin kappa constant | -2.3027578 |
| A_23_P161563 | MM_022337 | RAB38 | RAB38, member RAS oncogene family" | -2.2882872 |
| A_23_P121657 | NM_005114 | HS3ST1 | heparan sulfate (glucosamine) 3-O-sulfotransferase 1 | -2.273005 |
| A_23_P48217 | NM_030817 | APOLD1 | apolipoprotein L domain containing 1 | -2.27031 |
| A_23_ P86012 | NM_001017402 | LAMB3 | laminin, beta 3" | -2.252338 |
| A_23_P160968 | NM_018891 | LAMC2 | laminin, gamma 2" | -2.2487942 |
| A_23_P134734 | NM_017786 | GOLSYN | Golgi-localized protein | -2.2441505 |
| A_23_P217901 | NM_001126312 | RP11-544M22.4 | KAT protein | -2.219194 |
| A_23_P88819 | NM_017458 | MVP | major vault protein | -2.210613 |
| A_23_P111126 | L06175 | HCP5 | HLA complex P5 | -2.203004 |
| A_23_P160720 | NM_018664 | BATF3 | basic leucine zipper transcription factor, ATF-like 3 | -2.202483 |
| A_24_P173754 | NM_030806 | Clorf21 | chromosome 1 open reading frame 21 | -2.202426 |
| A_24_P388786 | NM_001369 | DNAH5 | dynein, axonemal, heavy chain 5 | -2.1940723 |
| A_23_P407096 | NM_152625 | ZNF366 | zinc finger protein 366 | -2.1921406 |
| A_23_P131935 | NM_017671 | FERMT1 | fermitin family homolog 1 (Drosophila) | -2.1760775 |
| A_23_P381645 | NM_001005463 | EBF3 | early B-cell factor 3 | -2.1604936 |
| A_23_P125705 | NM_021963 | NAP1L2 | nucleosome assembly protein 1-like 2 | -2.159929 |
| A_23_P422350 | NM_000260 | MYO7A | myosin VIIA | -2.1547 |
| A_23_P398476 | NM_022658 | HOXC8 | homeobox C8 | -2.1503644 |
| A_23_P210581 | NM_ 002237 | KCNG1 | potassium voltage-gated channel, subfamily G, member 1" | -2.144751 |
| A_32_P206415 | NM_001008781 | FAT 3 | FAT tumor suppressor homolog 3 (Drosophila) | -2.1277075 |
| A_23_P149121 | NM_004675 | DIRAS3 | DIRAS family, GTP-binding RAS-like 3 | -2.125239 |
| A_24_P944588 | NM_ 033196 | ZNF682 | zinc finger protein 682 | -2.1232018 |
| A_23_P171132 | NM_021783 | EDA2R | ectodysplasin A2 receptor | -2.1180549 |
| A_23_P104555 | NM_020349 | ANKRD2 | ankyrin repeat domain 2 (stretch responsive muscle) | -2.112261 |
| A_23_P69206 | NM_ 003043 | SLC6A6 | solute carrier family 6 (neurotransmitter transporter, taurine), member 6" | -2.110186 |
| A_23_P145054 | NM_001085480 | FAM162B | family with sequence similarity 162, member B" | -2.1101041 |
| A_23_P40217 | NM_018431 | DOK5 | docking protein 5 | -2.109531 |
| A_23_P139123 | NM_000062 | SERPING1 | serpin peptidase inhibitor, clade G (C1 inhibitor), member 1" | -2.098983 |
| A_24_P95059 | NM_007155 | ZP3 | zona pellucida glycoprotein 3 (sperm receptor) | -2.097852 |
| A_23_P48438 | NM_199162 | ADPRHL1 | ADP-ribosylhydrolase like 1 | -2.097145 |
| A_23_ P94319 | NM_014867 | KBTBD11 | kelch repeat and BTB (POZ) domain containing 11 | -2.087171 |
| A_23_ P160214 | NM_001080494 | TTC39A | tetratricopeptide repeat domain 39A | -2.084316 |
| A_24_P291231 | NM_ 016831 | PER3 | period homolog 3 (Drosophila) | -2.0800868 |
| A_23_P101623 | NM_022103 | ZNF667 | zinc finger protein 667 | -2.0795908 |
| A_23_P207221 | NM_018242 | SLC47A1 | solute carrier family 47, member 1" | -2.0635317 |
| A_24_P81789 | NM_019034 | RHOF | ras homolog gene family, member F (in filopodia) | -2.055134 |
| A_23_P85952 | NM_024901 | DENND2D | DENN/MADD domain containing 2D | -2.0337004 |
| A_23_ P16953 | NM_000867 | HTR2B | 5-hydroxytryptamine (serotonin) receptor 2B | -2.033488 |
| A_23_ P76538 | NM_017899 | TESC | tescalcin | -2.0306527 |
| A_23_P381431 | NM_030769 | NPL | N-acetylneuraminate pyruvate lyase (dihydrodipicolinate synthase) | -2.0289607 |
| A_23_P145024 | NM_ 000024 | ADRB2 | adrenergic, beta-2-, receptor, surface" | -2.0257697 |
| A_23_P169351 | NM_003026 | SH3GL2 | SH3-domain GRB2-like 2 | -2.0247844 |
| A_23_P152047 | NM_138967 | SCAMP5 | secretory carrier membrane protein 5 | -2.023881 |
| A_23_P388168 | NM_ 002867 | RAB3B | RAB3B, member RAS oncogene family" | -2.0154913 |
| A_23_P201497 | NM_182663 | RASSF5 | Ras association (RalGDS/AF-6) domain family member 5 | -2.0139146 |
| A_24_P925342 | AB209275 | MAN1C1 | mannosidase, alpha, class 1C, member 1 | -2.004328 |
| A_23_P130376 | NM_022068 | FAM38B | family with sequence similarity 38, member B | -2.000109 |

**Supplementary Table 4- Up-regulated genes in the microarray.**

| | | | Co-culture 6 days versus Mono-culture 6 days | |
|---|---|---|---|---|
| Unique ID | Target ID | Gene Symbol | Gene Name | M Value |
| A_24_P940694 | AK091400 | SLC44A5 | solute carrier family 44, member 5 | 2.0041642 |
| A_24_P171141 | AF130049 | LOC114227 | hypothetical protein LOC114227 | 2.008274 |
| A_23_P393401 | NR_003610 | PDXDC2 | pyridoxal-dependent decarboxylase domain containing 2 | 2.0142204 |
| A_23_P80570 | NM_001086 | AADAC | arylacetamide deacetylase | 2.0229129 |
| A_23_P81721 | NM_004277 | SLC25A27 | solute carrier family 25, member 27" | 2.0271153 |
| A_24_P419087 | NM_006576 | AVIL | advillin | 2.0292216 |
| A_23_P360542 | NR_023925 | C18orf2 | chromosome 18 open reading frame 2 | 2.037899 |
| A_23_P346048 | NR_002824 | HERC2P2 | hect domain and RLD 2 pseudogene 2 | 2.041409 |
| A_23_P60811 | NM_006252 | PRKAA2 | protein kinase, AMP-activated, alpha 2 catalytic subunit" | 2.0519602 |
| A_24_P222237 | NR_002824 | HERC2P2 | hect domain and RLD 2 pseudogene 2 | 2.065124 |
| A_23_P207493 | NM_016424 | CROP | cisplatin resistance-associated overexpressed protein | 2.070006 |
| A_32_P110550 | AB042555 | PDE4DIP | phosphodiesterase 4D interacting protein | 2.0739637 |
| A_23_P340308 | NM_176888 | TAS2R48 | taste receptor, type 2, member 48 | 2.0754272 |
| A_24_P60217 | AK055730 | SLC23A3 | solute carrier family 23 (nucleobase transporters), member 3" | 2.0777667 |
| A_24_P940725 | AL080186 | SFRS18 | splicing factor, arginine/serine-rich 18 | 2.0808406 |
| A_24_P98161 | NM_194455 | KRIT1 | KRIT1, ankyrin repeat containing | 2.0865297 |
| A_23_P28246 | NM_144712 | SLC23A3 | solute carrier family 23 (nucleobase transporters), member 3" | 2.0946742 |
| A_23_P54447 | BC069765 | C15orf5 | chromosome 15 open reading frame 5 | 2.0985755 |
| A_24_P453855 | AK126267 | PNPLA7 | patatin-like phospholipase domain containing 7 | 2.108414 |
| A_32_P169491 | AK098200 | LOC161527 | hypothetical protein LOC161527 | 2.1091287 |
| A_24_P341985 | NM_031938 | BCO2 | beta-carotene oxygenase 2 | 2.1149247 |
| A_32_P468289 | AL162056 | DOPEY 1 | dopey family member 1 | 2.1163766 |
| A_24_P50368 | NM_ 001001786 | BLID | BH3-like motif containing, cell death inducer" | 2.1165517 |
| A_24_P928522 | AK025142 | DST | dystonin | 2.126594 |
| A_32_P216872 | BX647358 | PDXDC2 | pyridoxal-dependent decarboxylase domain containing 2 | 2.1297436 |
| A_23_P303851 | NM_ 176886 | TAS2R45 | taste receptor, type 2, member 45 | 2.1319761 |
| A_32_P211080 | NR_ 002824 | HERC2P2 | hect domain and RLD 2 pseudogene 2 | 2.141752 |
| A_23_P75071 | NM_ 016195 | KIF20B | kinesin family member 20B | 2.1492891 |
| A_23_P354308 | AK025204 | ABI3BP | ABI family, member 3 (NESH) binding protein" | 2.1495505 |
| A_24_P8200 | AB095943 | SHPRH | SNF2 histone linker PHD RING helicase | 2.1648123 |
| A_24_P93754 | AB018323 | JMJD2C | jumonji domain containing 2C | 2.166888 |
| A_23_P257164 | NM_000481 | AMT | aminomethyltransferase | 2.169648 |
| A_32_P139738 | NR_002827 | HERC2P4 | hect domain and RLD 2 pseudogene 4 | 2.171488 |
| A_24_P925158 | D26122 | SF1 | splicing factor 1 | 2.1740255 |
| A_32_P78385 | BC094802 | DPY19L2P2 | dpy-19-like 2 pseudogene 2 (C. elegans) | 2.1745228 |
| A_23_P306511 | BC022302 | CMAH | cytidine monophosphate-N-acetylneuraminic acid hydroxylase (CMP-N-acetylneuraminate monooxygenase) pseudogene | 2.1938505 |
| A_23_P36865 | NM_025114 | CEP290 | centrosomal protein 290kDa | 2.1972585 |
| A_32_P48244 | ENST0000035829 6 | ZNF100 | zinc finger protein 100 | 2.2173142 |
| A_24_P932416 | NM_001123228 | TMEM14E | transmembrane protein 14E | 2.227468 |
| A_23_P31681 | AK074467 | C8orf38 | chromosome 8 open reading frame 38 | 2.2299826 |
| A_23_P8961 | NM_000880 | IL7 | interleukin 7 | 2.23187 |
| A_32_P195850 | MM_173812 | DPY19L2 | dpy-19-like 2 (C. elegans) | 2.2422097 |
| A_24_P910580 | NM_181077 | GOLGA8A | golgi autoantigen, golgin subfamily a, 8A" | 2.2559056 |
| A_24_P463973 | AK123878 | MEG3 | maternally expressed 3 (non-protein coding) | 2.2698766 |
| A_ 32_ P75559 | AK303593 | BST2 | bone marrow stromal cell antigen 2 | 2.272775 |
| A_23_P253622 | AK024934 | ANKRD36B | ankyrin repeat domain 36B | 2.291946 |
| A_23_P340312 | NM_176888 | TAS2R48 | taste receptor, type 2, member 48 | 2.2991645 |
| A_23_P115192 | NM_031282 | FCRL4 | Fc receptor-like 4 | 2.3092101 |
| A_24_P51067 | NM_ 025114 | CEP290 | centrosomal protein 290kDa | 2.3149905 |
| A_24_P265177 | AK022791 | PHC3 | polyhomeotic homolog 3 (Drosophila) | 2.3190363 |
| A_32_P156373 | XM_001715393 | LOC10013221 8 | hypothetical protein LOC100132218 | 2.3305676 |
| A_32_P208733 | AK055279 | UTP23 | UTP23, small subunit (SSU) processome component, homolog (yeast)" | 2.3333396 |
| A_24_P114249 | NM_004482 | GALNT3 | UDP-N-acetyl-alpha-D-galactosamine:polypeptide N-acetylgalactosaminyltransferase 3 (GalNAc-T3) | 2.3560943 |
| A_23_P374250 | NM_173812 | DPY19L2 | dpy-19-like 2 (C. elegans) | 2.3676346 |
| A_24_P687305 | NR_024583 | DKFZp434K19 1 | POM121 membrane glycoprotein-like 1 pseudogene | 2.3832968 |
| A_23_P426305 | NM_ 003734 | AOC3 | amine oxidase, copper containing 3 (vascular adhesion protein 1)" | 2.4014171 |
| A_23_ P331072 | BX647210 | LRRIQ3 | leucine-rich repeats and IQ motif containing 3 | 2.4346236 |
| A_23_P125748 | NM_032441 | ZMAT1 | zinc finger, matrin type 1" | 2.438655 |
| A_23_P37623 | NM_181077 | GOLGA8A | golgi autoantigen, golgin subfamily a, 8A" | 2.445613 |
| A_24_P128442 | NM_152380 | TBX15 | T-box 15 | 2.4838432 |
| A_32_P776863 | NM_173649 | C2orf61 | chromosome 2 open reading frame 61 | 2.4990112 |
| A_23_P52121 | NM_ 002614 | PDZK1 | PDZ domain containing 1 | 2.5010229 |
| A_23_P253524 | NM_001813 | CENPE | centromere protein E, 312kDa" | 2.541844 |
| A_23_P385911 | ENST0000039679 1 | KIAA1712 | KIAA1712 | 2.5994333 |
| A_23_P124805 | AK001243 | VPS13C | vacuolar protein sorting 13 homolog C (S. cerevisiae) | 2.6165174 |
| A_32_P122136 | AK057596 | LOC150759 | hypothetical protein LOC150759 | 2.667798 |
| A_24_P246841 | NM_004277 | SLC25A27 | solute carrier family 25, member 27" | 2.7717525 |
| A_23_P23611 | NM_001008219 | AMY1C | amylase, alpha 1C (salivary) | 2.7829946 |
| A_23_P353614 | NM_152765 | C8orf46 | chromosome 8 open reading frame 46 | 2.801072 |
| A_24_P916797 | AK000270 | AKAP9 | A kinase (PRKA) anchor protein (yotiao) 9 | 2.8275501 |
| A_24_P303420 | AK126092 | LOC221442 | hypothetical LOC221442 | 2.8380903 |
| A_24_P391591 | AK057596 | LOC150759 | hypothetical protein LOC150759 | 2.871928 |
| A_24_P11100 | NM_032441 | ZMAT1 | zinc finger, matrin type 1" | 2.8772666 |
| A_23_P51587 | NM_ 002924 | RGS7 | regulator of G-protein signaling 7 | 2.9475363 |
| A_23_P414793 | NM_000096 | CP | ceruloplasmin (ferroxidase) | 2.9993575 |
| A_24_P85258 | NM_001080484 | KIAA1751 | KIAA1751 | 3.0383245 |
| A_24_P344890 | AK095605 | AMY2B | amylase, alpha 2B (pancreatic) | 3.0388725 |
| A_23_P302060 | NM_176891 | IFNE | interferon, epsilon" | 3.0941112 |
| A_24_P53595 | NM_016592 | GNAS | GNAS complex locus | 3.2041835 |
| A_24_P310256 | NM_ 139284 | LGI4 | leucine-rich repeat LGI family, member 4" | 3.3231205 |
| A_24_P332081 | AL832756 | JAKMIP3 | janus kinase and microtubule interacting protein 3 | 3.339777 |
| A_23_P203191 | NM_000039 | APOA1 | apolipoprotein A-I | 3.3997842 |
| A_24_P341000 | AK092698 | FLJ35379 | similar to Alu subfamily J sequence contamination warning entry | 3.597919 |
| A_32_P9941 | NM_ 007191 | WIF1 | WNT inhibitory factor 1 | 3.8778475 |
| A_32_P197561 | NM_024007 | EBF1 | early B-cell factor 1 | 4.4216269 |

| Co-culture 6 days versus Co-culture 3 days | | | | |
|---|---|---|---|---|
| Unique ID | Target ID | Gene Symbol | Gene Name | M Value |
| A_24_P500584 | NR_ 001564 | XIST | X (inactive)-specific transcript (non-protein coding) | 12.5931163 |
| A_23_P155786 | NM_005420 | SULT1E1 | sulfotransferase family 1E, estrogen-preferring, member 1" | 8.2046563 |
| A_23_P95790 | NM_017625 | ITLN1 | intelectin 1 (galactofuranose binding) | 8.0198263 |
| A_23_P60130 | NM_052886 | MAL2 | mal, T-cell differentiation protein 2" | 7.5120488 |
| A_32_P179138 | NM_001130683 | GUCY1A3 | guanylate cyclase 1, soluble, alpha 3" | 6.6513906 |
| A_23_P350005 | NM_173553 | TRIML2 | tripartite motif family-like 2 | 5.6112492 |
| A_23_ P43164 | NM_015170 | SULF1 | sulfatase 1 | 5.589013 |
| A_24_ P213161 | NM_017852 | NLRP2 | NLR family, pyrin domain containing 2" | 5.5529553 |
| A_23_P129085 | NM_145658 | SPESP1 | sperm equatorial segment protein 1 | 5.2953218 |
| A_23_P69573 | NM_000856 | GUCY1A3 | guanylate cyclase 1, soluble, alpha 3" | 5.2366296 |
| A_24_P53778 | NM_080878 | ITLN2 | intelectin 2 | 5.2284345 |
| A_24_P75917 | NM_182568 | CCDC144B | coiled-coil domain containing 144B | 5.1691916 |
| A_32_P154911 | NM_175887 | PRR15 | proline rich 15 | 5.15205 |
| A_23_P67847 | NM_024572 | GALNT14 | UDP-N-acetyl-alpha-D-galactosamine:polypeptide N-acetylgalactosaminyltransferase 14 (GalNAc-T14) | 4.879263 |
| A_24_ P13041 | NM_145307 | RTKN2 | rhotekin 2 | 4.6814901 |
| A_23_P155688 | NM_021114 | SPINK2 | serine peptidase inhibitor, Kazal type 2 (acrosin-trypsin inhibitor) | 4.66596 |
| A_24_P288915 | AK093811 | CCDC144B | coiled-coil domain containing 144B | 4.6400898 |
| A_23_P371729 | NM_005266 | GJA5 | gap junction protein, alpha 5, 40kDa" | 4.6273297 |
| A_23_P15450 | NM_018286 | TMEM100 | transmembrane protein 100 | 4.5045573 |
| A_23_P36531 | NM_004616 | TSPAN8 | tetraspanin 8 | 4.4025021 |
| A_23_P403445 | NM_006569 | CGREF1 | cell growth regulator with EF-hand domain 1 | 4.239469 |
| A_23_P56746 | NM_004460 | FAP | fibroblast activation protein, alpha" | 4.1927154 |
| A_24_P288890 | NM_181709 | FAM101A | family with sequence similarity 101, member A | 4.065982 |
| A_23_P52410 | NM_145307 | RTKN2 | rhotekin 2 | 4.0503195 |
| A_23_P95029 | NM_021021 | SNTB1 | syntrophin, beta 1 (dystrophin-associated protein A1, 59kDa, basic component 1)" | 4.0177255 |
| A_23_P406385 | NM_153350 | FBXL16 | F-box and leucine-rich repeat protein 16 | 4.0115285 |
| A_23_ P105144 | NM_020974 | SCUBE2 | signal peptide, CUB domain, EGF-like 2 | 4.007575 |
| A_32_P200697 | NM_181709 | FAM101A | family with sequence similarity 101, member A | 3.966588 |
| A_23_P215459 | NM_000501 | ELN | elastin | 3.9293356 |
| A_23_P58676 | NM_024563 | C5orf23 | chromosome 5 open reading frame 23 | 3.8902445 |
| A_23_P217917 | NM_147148 | GSTM4 | glutathione S-transferase mu 4 | 3.8893845 |
| A_32_P181077 | NM_203447 | DOCK8 | dedicator of cytokinesis 8 | 3.8843326 |
| A_23_P257649 | NM_002899 | RBP1 | retinol binding protein 1, cellular" | 3.8630243 |
| A_23_P25353 | NM_000908 | NPR3 | natriuretic peptide receptor C/guanylate cyclase C (atrionatriuretic peptide receptor C) | 3.7955647 |
| A_23_P69497 | NM_003278 | CLEC3B | C-type lectin domain family 3, member B" | 3.7919 |
| A_23_P327451 | NM_000908 | NPR3 | natriuretic peptide receptor C/guanylate cyclase C (atrionatriuretic peptide receptor C) | 3.7844863 |
| A_23_P421401 | NM_002609 | PDGFRB | platelet-derived growth factor receptor, beta polypeptide" | 3.7825935 |
| A_23_P19369 | NM_017640 | LRRC16A | leucine rich repeat containing 16A | 3.7317835 |
| A_24_P164505 | BC098294 | FAM106A | family with sequence similarity 106, member A" | 3.7136457 |
| A_24_P639679 | AK095831 | SNORD123 | small nucleolar RNA, C/D box 123" | 3.660561 |
| A_24_P369232 | NM_031455 | CCDC3 | coiled-coil domain containing 3 | 3.6385524 |
| A_23_P69738 | NM_023940 | RASL11B | RAS-like, family 11, member B | 3.5668816 |
| A_24_P738168 | ENST0000032979 8 | FREM3 | FRAS1 related extracellular matrix 3 | 3.5534517 |
| A_23_P31273 | NM_001635 | AMPH | amphiphysin | 3.5149605 |
| A_23_P132956 | NM_004181 | UCHL1 | ubiquitin carboxyl-terminal esterase L1 (ubiquitin thiolesterase) | 3.496619 |
| A_23_P93737 | NM_004411 | DYNC1I1 | dynein, cytoplasmic 1, intermediate chain 1" | 3.4706085 |
| A_23_P121926 | NM_005410 | SEPP1 | selenoprotein P, plasma, 1" | 3.439935 |
| A_23_P63736 | BC007394 | MGC16291 | hypothetical protein MGC16291 | 3.4054182 |
| A_23_P144911 | NM_152403 | EGFLAM | EGF-like, fibronectin type III and laminin G domains | 3.3878002 |
| A_23_P31121 | NM_002996 | CX3CL1 | chemokine (C-X3-C motif) ligand 1 | 3.38521 |
| A_23_P49391 | NM_016212 | TP53TG3 | TP53 target 3 | 3.3187182 |
| A_23_P79251 | NM_014600 | EHD3 | EH-domain containing 3 | 3.2961135 |
| A_23_P386030 | AF028828 | SNTB1 | syntrophin, beta 1 (dystrophin-associated protein A1, 59kDa, basic component 1)" | 3.2353347 |
| A_23_P83098 | NM_000689 | ALDH1A1 | aldehyde dehydrogenase 1 family, member A1" | 3.212209 |
| A_23_P61967 | NM_014469 | RBMXL2 | RNA binding motif protein, X-linked-like 2" | 3.1963621 |
| A_23_P47579 | NM_176822 | NLRP14 | NLR family, pyrin domain containing 14 | 3.1881834 |
| A_24_P212021 | NM_000851 | GSTM5 | glutathione S-transferase mu 5 | 3.18169 |
| A_23_P83134 | NM_002048 | GAS1 | growth arrest-specific 1 | 3.1669307 |
| A_23_P79272 | NM_001003683 | PDE1A | phosphodiesterase 1A, calmodulin-dependent" | 3.161868 |
| A_23_ P66827 | AK021862 | FAM106A | family with sequence similarity 106, member A | 3.1335342 |
| A_24_P118196 | NM_001080393 | GLT8D4 | glycosyltransferase 8 domain containing 4 | 3.1078747 |
| A_23_P92983 | NM_ 017614 | BHMT2 | betaine-homocysteine methyltransferase 2 | 3.1073714 |
| A_32_P191840 | XM_933903 | LOC644662 | similar to hCG2042541 | 3.0930806 |
| A_32_P16007 | NM_ 207355 | POTEB | POTE ankyrin domain family, member B" | 3.0563867 |
| A_32_P60065 | NM_004101 | F2RL2 | coagulation factor II (thrombin) receptor-like 2 | 3.017839 |
| A_23_P144718 | NM_004101 | F2RL2 | coagulation factor II (thrombin) receptor-like 2 | 2.988743 |
| A_23_P407497 | NM_013959 | NRG1 | neuregulin 1 | 2.971679 |
| A_24_P270728 | NM_001042483 | NUPR1 | nuclear protein 1 | 2.9712219 |
| A_23_P33326 | NM_ 000679 | ADRA1B | adrenergic, alpha-1B-, receptor" | 2.9130255 |
| A_23 _P207003 | NM_004574 | 38231 | septin 4 | 2.9109967 |
| A_23_P420348 | NM_ 174981 | POTED | POTE ankyrin domain family, member D" | 2.9017281 |
| A_23_P167030 | NM_ 000316 | PTH1R | parathyroid hormone 1 receptor | 2.8829803 |
| A_23_P54144 | NM_ 001202 | BMP4 | bone morphogenetic protein 4 | 2.87191 |
| A_23_P151805 | NM_006329 | FBLN5 | fibulin 5 | 2.868258 |
| A_32_P471485 | BC025765 | RTKN2 | rhotekin 2 | 2.8634051 |
| A_23_P333029 | NM_173549 | C8orf47 | chromosome 8 open reading frame 47 | 2.8633264 |
| A_23_P26890 | NM_024302 | MMP28 | matrix metallopeptidase 28 | 2.8536377 |
| A_23_P56328 | NM_031310 | PLVAP | plasmalemma vesicle associated protein | 2.840474 |
| A_32_P109214 | NM_001004306 | MGC87631 | similar to hypothetical protein FLJ36492 | 2.833538 |
| A_23_P113351 | NM_004684 | SPARCL1 | SPARC-like 1 (hevin) | 2.8273755 |
| A_23_P155596 | MM_001002294 | FMO3 | flavin containing monooxygenase 3 | 2.8262236 |
| A_23_P114883 | NM_002023 | FMOD | fibromodulin | 2.8169279 |
| A_24_P220485 | NM_182487 | OLFML2A | olfactomedin-like 2A | 2.8071563 |
| A_24_P943588 | AF201385 | TXNRD2 | thioredoxin reductase 2 | 2.7889967 |
| A_24_P246196 | MM_214675 | CLEC4M | C-type lectin domain family 4, member M" | 2.7829864 |
| A_23_P302672 | NM_145244 | DDIT4L | DNA-damage-inducible transcript 4-like | 2.780097 |
| A_23_P75769 | NM_024021 | MS4A4A | membrane-spanning 4-domains, subfamily A, member 4 | 2.7788735 |
| A_23_P64785 | MM_152320 | ZNF641 | zinc finger protein 641 | 2.771647 |
| A_24_P292849 | AL137382 | LOC146429 | hypothetical protein LOC146429 | 2.7697638 |
| A_32_P50066 | MM_001039580 | MAP9 | microtubule-associated protein 9 | 2.764735 |
| A_23_P144916 | NM_ 005110 | GFPT2 | glutamine-fructose-6-phosphate transaminase 2 | 2.745744 |
| A_23_P422831 | NM_004816 | C9orf61 | chromosome 9 open reading frame 61 | 2.739364 |
| A_23_P428080 | AB020701 | KIAA0894 | KIAA0894 protein | 2.7391762 |
| A_23_P302568 | NM_003459 | SLC30A3 | solute carrier family 30 (zinc transporter), member 3 | 2.7326405 |
| A_23_P214168 | NM_ 004370 | COL12A1 | collagen, type XII, alpha 1" | 2.724168 |
| A_23_P122924 | NM_002192 | INHBA | inhibin, beta A" | 2.7137955 |
| A_23_P29124 | NM_002688 | 38596 | septin 5 | 2.713282 |
| A_23_P360534 | NR_023925 | C18orf2 | chromosome 18 open reading frame 2 | 2.7031812 |
| A_23_P214803 | NM_014841 | SNAP91 | synaptosomal-associated protein, 91kDa homolog (mouse) | 2.7022004 |
| A_32_P32413 | NM_015559 | SETBP1 | SET binding protein 1 | 2.695718 |
| A_24_P291814 | NM_ 004370 | COL12A1 | collagen, type XII, alpha 1" | 2.6940175 |
| A_24_P208436 | NM_001003683 | PDE1A | phosphodiesterase 1A, calmodulin-dependent | 2.6746507 |
| A_23_P51587 | NM_002924 | RGS7 | regulator of G-protein signaling 7 | 2.6739832 |
| A_23_P414793 | NM_000096 | CP | ceruloplasmin (ferroxidase) | 2.6516814 |
| A_23_P110473 | NM_004536 | NAIP | NLR family, apoptosis inhibitory protein" | 2.6419156 |
| A_23_P138655 | NM_057157 | CYP26A1 | cytochrome P450, family 26, subfamily A, polypeptide 1" | 2.641847 |
| A_23_P215744 | NM_033427 | CTTNBP2 | cortactin binding protein 2 | 2.6312279 |
| A_24_P712271 | NM_207328 | LOC150763 | hypothetical protein LOC150763 | 2.6251041 |
| A_24_P221414 | NM_004411 | DYNC1I1 | dynein, cytoplasmic 1, intermediate chain 1" | 2.6146931 |
| A_23_ P372974 | NM_152402 | TRAM1L1 | translocation associated membrane protein 1-like 1 | 2.6134994 |
| A_24_P222237 | NR_002824 | HERC2P2 | hect domain and RLD 2 pseudogene 2 | 2.599507 |
| A_23_P116642 | NM_133489 | SLC26A10 | solute carrier family 26, member 10" | 2.5993607 |
| A_23_P87879 | NM_001781 | CD69 | CD69 molecule | 2.5931544 |
| A_23_P4551 | NM_015559 | SETBP1 | SET binding protein 1 | 2.592962 |
| A_23_P8820 | NM_001442 | FABP4 | fatty acid binding protein 4, adipocyte" | 2.589049 |
| A_32_P211080 | NR_002824 | HERC2P2 | hect domain and RLD 2 pseudogene 2 | 2.584551 |
| A_23_P371495 | NM_175861 | TMTC1 | transmembrane and tetratricopeptide repeat containing 1 | 2.5771015 |
| A_24_P218805 | NM_017409 | HOXC10 | homeobox C10 | 2.5694865 |
| A_23_P121564 | NM_000857 | GUCY1B3 | guanylate cyclase 1, soluble, beta 3 | 2.558513 |
| A_23_P126075 | NM_002245 | KCNK1 | potassium channel, subfamily K, member 1" | 2.5456856 |
| A_24_P356916 | NM_001011554 | SLC13A3 | solute carrier family 13 (sodium-dependent dicarboxylate transporter), member 3" | 2.5359893 |
| A_24_P53465 | NM_214675 | CLEC4M | C-type lectin domain family 4, member M" | 2.5326324 |
| A_23_ P152305 | NM_ 001797 | CDH11 | cadherin 11, type 2, OB-cadherin (osteoblast)" | 2.519575 |
| A_24_P184803 | NM_004086 | COCH | coagulation factor C homolog, cochlin (Limulus polyphemus)" | 2.5007844 |
| A_32_P34444 | NM_025135 | FHOD3 | formin homology 2 domain containing 3 | 2.5001057 |
| A_23_P357571 | NM_000854 | GSTT2 | glutathione S-transferase theta 2 | 2.490856 |
| A_23_P346048 | NR_002824 | HERC2P2 | hect domain and RLD 2 pseudogene 2 | 2.479499 |
| A_23_P346093 | NM_152468 | TMC8 | transmembrane channel-like 8 | 2.4763698 |
| A_23_P166797 | NM_022147 | RTP4 | receptor (chemosensory) transporter protein 4 | 2.4744943 |
| A_23_P419696 | NM_144586 | LYPD1 | LY6/PLAUR domain containing 1 | 2.4715535 |
| A_23_P204286 | NM_000900 | MGP | matrix Gla protein | 2.468325 |
| A_32_P139738 | NR_ 002827 | HERC2P4 | hect domain and RLD 2 pseudogene 4 | 2.457105 |
| A_23_ P106933 | NM_052956 | ACSM1 | acyl-CoA synthetase medium-chain family member 1 | 2.4475286 |
| A_23_P904 | NM_024603 | BEND5 | BEN domain containing 5 | 2.4432084 |
| A_23_P115161 | NM_ 002036 | DARC | Duffy blood group, chemokine receptor" | 2.4372559 |
| A_23_P27013 | NM_024017 | HOXB9 | homeobox B9 | 2.4330936 |
| A_23_P142239 | AK027130 | LOC541469 | hypothetical protein LOC541469 | 2.4050138 |
| A_24_P221327 | BC020847 | LOC644246 | hypothetical protein LOC644246 | 2.395498 |
| A_23_P28334 | NM_ 003853 | IL18RAP | interleukin 18 receptor accessory protein | 2.333673 |
| A_23_P259442 | NM_001873 | CPE | carboxypeptidase E | 2.330546 |
| A_23_P302634 | BC101016 | C12orf64 | chromosome 12 open reading frame 64 | 2.3142719 |
| A_23_P50697 | NM_ 006905 | PSG1 | pregnancy specific beta-1-glycoprotein 1 | 2.3101452 |
| A_24_P579826 | BC071681 | ARL17 | ADP-ribosylation factor-like 17 | 2.3073995 |
| A_23_P258769 | NM_002121 | HLA-DPB1 | major histocompatibility complex, class II, DP beta 1" | 2.3068786 |
| A_23_P52761 | NM_002423 | MMP7 | matrix metallopeptidase 7 (matrilysin, uterine)" | 2.3065215 |
| A_23_P257993 | NM_004944 | DNASE1L3 | deoxyribonuclease I-like 3 | 2.304541 |
| A_23_P34126 | NM_001711 | BGN | biglycan | 2.295382 |
| A_23_P19020 | NM_005460 | SNCAIP | synuclein, alpha interacting protein" | 2.294381 |
| A_24_P331830 | NM_015209 | RP1-21018.1 | kazrin | 2.286087 |
| A_23_P337346 | AK056484 | hCG_20099 21 | hypothetical locus LOC441204 | 2.2788632 |
| A_23_P13094 | NM_002425 | MMP10 | matrix metallopeptidase 10 (stromelysin 2) | 2.2774393 |
| A_23_P6818 | NM_020163 | SEMA3G | sema domain, immunoglobulin domain (Ig), short basic domain, secreted, (semaphorin) 3G | 2.2771295 |
| A_24_P128442 | NM_152380 | TBX15 | T-box 15 | 2.2662993 |
| A_24_P246406 | NM_001006605 | FAM69A | family with sequence similarity 69, member A" | 2.2629583 |
| A_23_P356494 | NM_006846 | SPINK5 | serine peptidase inhibitor, Kazal type 5" | 2.2532257 |
| A_24_P64401 | BC007394 | MGC16291 | hypothetical protein MGC16291 | 2.2515278 |
| A_24_P940694 | AK091400 | SL1C44A5 | solute carrier family 44, member 5 | 2.250309 |
| A_23_P206920 | NM_001040114 | MYH11 | myosin, heavy chain 11, smooth muscle | 2.2377084 |
| A_23_P16252 | NM_002257 | KLK1 | kallikrein 1 | 2.2310541 |
| A_24_P231829 | NM_017614 | BHMT2 | betaine-homocysteine methyltransferase 2 | 2.2256352 |
| A_24_P59799 | NM_024781 | CCDC102B | coiled-coil domain containing 102B | 2.2234418 |
| A_23_P109427 | NM_000854 | GSTT2 | glutathione S-transferase theta 2 | 2.21082 |
| A_24_P245589 | NM_031310 | PLVAP | plasmalemma vesicle associated protein | 2.2015346 |
| A_23_P256033 | NM_001958 | EEF1A2 | eukaryotic translation elongation factor 1 alpha 2 | 2.1877389 |
| A_23_P357207 | NM_138409 | MRAP2 | melanocortin 2 receptor accessory protein 2 | 2.182037 |
| A_32_P2452 | NM_175861 | TMTC1 | transmembrane and tetratricopeptide repeat containing 1 | 2.174506 |
| A_23_P30075 | NM_006095 | ATP8A1 | ATPase, aminophospholipid transporter (APLT), class I, type 8A, member 1" | 2.1730165 |
| A_23_ P423074 | NM_015566 | FAM169A | family with sequence similarity 169, member A" | 2.1701266 |
| A_24_P208595 | NM_053034 | ANTXR1 | anthrax toxin receptor 1 | 2.1540107 |
| A_24_P273799 | ENST0000030104 2 | ZNF641 | zinc finger protein 641 | 2.14752 |
| A_23_P370027 | AK124788 | GGT7 | gamma-glutamyltransferase 7 | 2.1393684 |
| A_24_P231010 | NM_018995 | MOV10L1 | Mov10l1, Moloney leukemia virus 10-like 1, homolog (mouse)" | 2.1283951 |
| A_23_P204847 | NM_002298 | LCP1 | lymphocyte cytosolic protein 1 (L-plastin) | 2.1257206 |
| A_23_P116898 | NM_000014 | A2M | alpha-2-macroglobulin | 2.124616 |
| A_ 32_ P24122 | NM_015894 | STMN3 | stathmin-like 3 | 2.118763 |
| A_24_P192485 | NM_002546 | TNFRSF11B | tumor necrosis factor receptor superfamily, member 11b" | 2.103257 |
| A_23_P39202 | NM_033520 | C19orf33 | chromosome 19 open reading frame 33 | 2.0971413 |
| A_24_P396662 | NM_147148 | GSTM4 | glutathione S-transferase mu 4 | 2.093116 |
| A_23_P122906 | NM_015570 | AUTS2 | autism susceptibility candidate 2 | 2.0852408 |
| A_23_P217319 | NM_004114 | FGF13 | fibroblast growth factor 13 | 2.0801625 |
| A_24_P380734 | NM_002998 | SDC2 | syndecan 2 | 2.077774 |
| A_24_P363408 | NM_012259 | HEY2 | hairy/enhancer-of-split related with YRPW motif 2 | 2.0719206 |
| A_23_P44794 | NM_138453 | RAB3C | RAB3C, member RAS oncogene family" | 2.0644617 |
| A_23_P305198 | NM_003151 | STAT4 | signal transducer and activator of transcription 4 | 2.0440189 |
| A_23_P203957 | NM_175861 | TMTC1 | transmembrane and tetratricopeptide repeat containing 1 | 2.039811 |
| A_23_P27795 | NM_021102 | SPINT2 | serine peptidase inhibitor, Kunitz type, 2" | 2.037252 |
| A_23_P372308 | NM_020211 | RGMA | RGM domain family, member A" | 2.034106 |
| A_32_P216566 | NM_001009994 | C6orf159 | chromosome 6 open reading frame 159 | 2.0275353 |
| A_24_P246573 | NM_015209 | RP1-21018.1 | kazrin | 2.0188723 |
| A_23_P349321 | NM_022166 | XYLT1 | xylosyltransferase I | 2.0100237 |
| A_24_P920525 | AK022468 | SORBS1 | sorbin and SH3 domain containing 1 | 2.007313 |

### REFERENCES

1. Cecchelli, R., et al. Modelling of the blood-brain barrier in drug discovery and development. Nat Rev Drug Discov 6, 650-661 (2007).
2. Weksler, B.B., et al. Blood-brain barrier-specific properties of a human adult brain endothelial cell line. FASEB J 19, 1872-1874 (2005).
3. Sano, Y., et al. Establishment of a new conditionally immortalized human brain microvascular endothelial cell line retaining an in vivo blood-brain barrier function. J Cell Physiol 225, 519-528 (2010).
4. Lippmann, E.S., et al. Derivation of blood-brain barrier endothelial cells from human pluripotent stem cells. Nat Biotechnol (2012).
5. Pedroso, D.C., et al. Improved survival, vascular differentiation and wound healing potential of stem cells co-cultured with endothelial cells. PLoS One 6, e16114 (2011).
6. Armulik, A., et al. Pericytes regulate the blood-brain barrier. Nature 468, 557-561 (2010).
7. Daneman, R., Zhou, L., Kebede, A.A. & Barres, B.A. Pericytes are required for blood-brain barrier integrity during embryogenesis. Nature 468, 562-566 (2010).
8. Vandenhaute, E., et al. Brain pericytes from stress-susceptible pigs increase blood-brain barrier permeability in vitro. Fluids Barriers CNS 9, 11 (2012).
9. Liebner, S., et al. Wnt/beta-catenin signaling controls development of the blood-brain barrier. J Cell Biol 183, 409-417 (2008).
10. Tatsuta, T., Naito, M., Oh-hara, T., Sugawara, I. & Tsuruo, T. Functional involvement of P-glycoprotein in blood-brain barrier. J Biol Chem 267, 20383-20391 (1992).
11. Bo, L., et al. Distribution of immunoglobulin superfamily members ICAM-1, -2, -3, and the beta 2 integrin LFA-1 in multiple sclerosis lesions. J Neuropathol Exp Neurol 55, 1060-1072 (1996).
12. Friden, M., Gupta, A., Antonsson, M., Bredberg, U. & Hammarlund-Udenaes, M. In vitro methods for estimating unbound drug concentrations in the brain interstitial and intracellular fluids. Drug Metab Dispos 35, 1711-1719 (2007).
13. Alvarez, J.I., et al. The Hedgehog pathway promotes blood-brain barrier integrity and CNS immune quiescence. Science 334, 1727-1731 (2011).
14. Daneman, R., et al. Wnt/beta-catenin signaling is required for CNS, but not non-CNS, angiogenesis. Proc Natl Acad Sci U S A 106, 641-646 (2009).
15. Booher J, Sensenbrenner M. Growth and cultivation of dissociated neurons and glial cells from embryonic chick, rat and human brain in flask cultures. Neurobiology. 1972;2(3):97-105.
16. Descamps L, Coisne C, Dehouck B, Cecchelli R, Torpier G. Protective effect of glial cells against lipopolysaccharide-mediated blood-brain barrier injury. Glia. 2003 Apr 1;42(1):46-58.
17. Meresse S, Dehouck MP, Delorme P, Bensaid M, Tauber JP, Delbart C, Fruchart JC, Cecchelli R. Bovine brain endothelial cells express tight junctions and monoamine oxidase activity in long-term culture. J Neurochem 53(5):1363-1371.1989.
18. Saeed AI, Bhagabati NK, Braisted JC, Liang W, Sharov V, Howe EA, Li J, Thiagarajan M, White JA, Qackenbush J. TM4 microarray suite software. Methods Enzymol. 2006(411): 134-193.
19. Sano Y, Shimizu F, Abe M, Maeda T, Kashiwamura Y, Ohtsuki S, Terasaki T, Obinata M, Kajiwara K, Fujii M, Suzuki M, Kanda T. Establisment of a new conditionally immortalized human brain microvascular endothelial cell line retaining an in vivo blood-brain barrier function. J Cell Physiol. 2010 (225): 519-528.
20. Siflinger-Birnboim A, Del Vecchio PJ, Cooper JA, Blumenstock FA, Shepard JM, Malik AB. Molecular sieving characteristics of the cultured endothelial monolayer. J Cell Physiol. 1987; 132(1):111-117.
21. Simon R, Lam A, Li MC, Ngan M, Menenzes S, Zhao Y. Analysis of gene expression data using BRB-array tools. Cancer Inform. 2007(3): 11-17.

## Claims

1. A method for obtaining human brain-like endothelial cells comprising the following steps:
contacting a population of cells isolated from CD34+ stem cells with a differentiation medium to obtain endothelial cells wherein the said stem cells are isolated from cord blood or peripheral blood;
co-culturing the said endothelial cells with pericytes or with a pericytes conditioned medium, wherein said pericytes express at least one of the following markers: vimentin, PDGF-β, NG-2, α-SMA, P-gp, γ-GT;
wherein the population of endothelial cells is co-cultured with pericytes or with the pericytes conditioned medium during at least 4 days.

2. The method according to any of the previous claims wherein the said differentiation medium is EGM-2 medium with 20% (v/v) FBS and 50 ng/mL of VEGF165.

3. The method according to any of the previous claims wherein the population of endothelial cells is co-cultured with pericytes during 5-6 days.

## Patentansprüche

1. Ein Verfahren zur Gewinnung menschlicher hirnähnlicher Endothelzellen, umfassend die folgenden Schritte:
in Kontakt bringen einer aus einem CD34+-Stamm isolierten Zellpopulation mit einem Differenzierungsmedium, um Endothelzellen zu erhalten, wobei die genannten Stammzellen aus Nabelschnurblut oder peripherem Blut isoliert werden;
Co-Kultivieren der genannten Endothelzellen mit Perizyten oder mit einem Perizyten-konditionierten Medium, wobei die genannten Perizyten mindestens einen der folgenden Marker exprimieren: Vimentin, PDGF-β, NG-2, α-SMA, P-gp, γ-GT;
wobei die Endothelzellen-Population während mindestens 4 Tagen mit Perizyten oder mit dem Perizyten-konditionierten Medium co-kultiviert wird.

2. Das Verfahren nach einem der vorangehenden Ansprüche, wobei das genannte Differenzierungsmedium ein EGM-2-Medium mit 20% (v/v) FBS und 50 ng/ml VEGF165 ist.

3. Das Verfahren nach einem der vorangehenden Ansprüche, wobei die Endothelzellen-Population während 5-6 Tagen mit Perizyten co-kultiviert wird.

## Revendications

1. Un procédé pour obtenir des cellules endothéliales de type encéphalique humaines comprenant les étapes suivantes :
mettre une population de cellules isolées de cellules souche CD34+ en contact avec un moyen de différenciation pour obtenir des cellules endothéliales dans lequel lesdites cellules souche sont isolées du sang ombilical ou du sang périphérique ;
co-cultiver lesdites cellules endothéliales avec des péricytes ou avec un moyen conditionné par des péricytes, dans lequel lesdits péricytes expriment au moins un des marqueurs suivants : vimentine, PDGF-β, NG-2, α-SMA, P-gp, γ-GT ;
dans lequel la population de cellules endothéliales est co-cultivée avec des péricytes ou avec le moyen conditionné de péricytes durant au moins 4 jours.

2. Le procédé selon l'une quelconque des revendications précédentes dans lequel ledit moyen de différentiation est le moyen EGM-2 avec 20% (v/v) FBS et 50 ng/mL de VEGF165.

3. Le procédé selon l'une quelconques des revendications précédentes dans lequel la population de cellules endothéliales est co-cultivée avec des péricytes durant 5-6 jours.


### SEQUENCE LISTING

<110> BIOCANT - CENTRO DE INOVAÇÃO EM BIOTECNOLOGIA ; UNIVERSITY OF ARTOIS
<120> A HUMAN BLOOD-BRAIN BARRIER MODEL DERIVED FROM STEM CELLS
<130> PPI 49333-14
<160> 60
<170> Patent In version 3.5
<210> 1
   <211> 20
   <212> DNA
   <213> Homo sapiens
<400> 1
   agccacatcg ctcagacacc 20
<210> 2
   <211> 18
   <212> DNA
   <213> Homo sapiens
<400> 2
   gaaagactac gtgtgaca 18
<210> 3
   <211> 18
   <212> DNA
   <213> Homo sapiens
<400> 3
   atcacgtcgc agaacatc 18
<210> 4
   <211> 20
   <212> DNA
   <213> Homo sapiens
<400> 4
   ttaacagacg gaatgaagtt 20
<210> 5
   <211> 22
   <212> DNA
   <213> Homo sapiens
<400> 5
   ttctggatct ctatatggtt ca 22
<210> 6
   <211> 20
   <212> DNA
   <213> Homo sapiens
<400> 6
   cctgaaccag tatctgataa 20
<210> 7
   <211> 24
   <212> DNA
   <213> Homo sapiens
<400> 7
   tgagagaatg agatttctgc ttgt 24
<210> 8
   <211> 18
   <212> DNA
   <213> Homo sapiens
<400> 8
   ttggctccaa ggaagatt 18
<210> 9
   <211> 18
   <212> DNA
   <213> Homo sapiens
<400> 9
   gagacacttg ccttcttc 18
<210> 10
   <211> 19
   <212> DNA
   <213> Homo sapiens
<400> 10
   ttgacaccac taagatgat 19
<210> 11
   <211> 18
   <212> DNA
   <213> Homo sapiens
<400> 11
   cctcctgaga catctttg 18
<210> 12
   <211> 18
   <212> DNA
   <213> Homo sapiens
<400> 12
   acacaaagcc aataagac 18
<210> 13
   <211> 18
   <212> DNA
   <213> Homo sapiens
<400> 13
   atgctgacaa taacacaa 18
<210> 14
   <211> 19
   <212> DNA
   <213> Homo sapiens
<400> 14
   atcctctgcc tcaaattct 19
<210> 15
   <211> 19
   <212> DNA
   <213> Homo sapiens
<400> 15
   cgggagatca agcagaatg 19
<210> 16
   <211> 18
   <212> DNA
   <213> Homo sapiens
<400> 16
   gcttcgtcaa gtgcaaca 18
<210> 17
   <211> 21
   <212> DNA
   <213> Homo sapiens
<400> 17
   tacctcacaa aacccccatc c 21
<210> 18
   <211> 21
   <212> DNA
   <213> Homo sapiens
<400> 18
   tcgtcagtac catatcccat g 21
<210> 19
   <211> 19
   <212> DNA
   <213> Homo sapiens
<400> 19
   gatgataacg gcgatgtga 19
<210> 20
   <211> 21
   <212> DNA
   <213> Homo sapiens
<400> 20
   ggagtcacag tgccatcaca t 21
<210> 21
   <211> 20
   <212> DNA
   <213> Homo sapiens
<400> 21
   aaggaacact gacatcaatt 20
<210> 22
   <211> 32
   <212> DNA
   <213> Homo sapiens
<400> 22
   gcctggcagc tggaagacaa atacacaaaa tt 32
<210> 23
   <211> 20
   <212> DNA
   <213> Homo sapiens
<400> 23
   tggctgtcat ggcttcagta 20
<210> 24
   <211> 21
   <212> DNA
   <213> Homo sapiens
<400> 24
   accaagacgt atcaggtggc c 21
<210> 25
   <211> 25
   <212> DNA
   <213> Homo sapiens
<400> 25
   ccaatctact ctcacttcag cgaga 25
<210> 26
   <211> 22
   <212> DNA
   <213> Homo sapiens
<400> 26
   aagtgaacaa cctccagttc ca 22
<210> 27
   <211> 22
   <212> DNA
   <213> Homo sapiens
<400> 27
   agtggcactg tcagatcaaa tt 22
<210> 28
   <211> 22
   <212> DNA
   <213> Homo sapiens
<400> 28
   ctgctatggg actattgctg tg 22
<210> 29
   <211> 20
   <212> DNA
   <213> Homo sapiens
<400> 29
   ctcgaatgga aactgaacac 20
<210> 30
   <211> 20
   <212> DNA
   <213> Homo sapiens
<400> 30
   gcatcctgat cgagcacctg 20
<210> 31
   <211> 19
   <212> DNA
   <213> Homo sapiens
<400> 31
   gtactcagcg ccagcatcg 19
<210> 32
   <211> 24
   <212> DNA
   <213> Homo sapiens
<400> 32
   ggtcctaatg ttaatgatag tatc 24
<210> 33
   <211> 20
   <212> DNA
   <213> Homo sapiens
<400> 33
   tacaccttgc actgcatctg 20
<210> 34
   <211> 18
   <212> DNA
   <213> Homo sapiens
<400> 34
   aagcgaaatc ctcagtct 18
<210> 35
   <211> 20
   <212> DNA
   <213> Homo sapiens
<400> 35
   ccacaacaca gtagtgatac 20
<210> 36
   <211> 19
   <212> DNA
   <213> Homo sapiens
<400> 36
   aatcttctca ctccttctg 19
<210> 37
   <211> 18
   <212> DNA
   <213> Homo sapiens
<400> 37
   tagggctcac agggatgg 18
<210> 38
   <211> 20
   <212> DNA
   <213> Homo sapiens
<400> 38
   gagtaaggtc cagaatgaca 20
<210> 39
   <211> 20
   <212> DNA
   <213> Homo sapiens
<400> 39
   gctttgtagt tcatagttcg 20
<210> 40
   <211> 18
   <212> DNA
   <213> Homo sapiens
<400> 40
   gtagcaatga ggttccaa 18
<210> 41
   <211> 18
   <212> DNA
   <213> Homo sapiens
<400> 41
   ctggaatatg acgggaag 18
<210> 42
   <211> 19
   <212> DNA
   <213> Homo sapiens
<400> 42
   acagaatcca acataggta 19
<210> 43
   <211> 18
   <212> DNA
   <213> Homo sapiens
<400> 43
   ccaagtagcc aatcataa 18
<210> 44
   <211> 18
   <212> DNA
   <213> Homo sapiens
<400> 44
   ttcgtctaac tccgttgg 18
<210> 45
   <211> 19
   <212> DNA
   <213> Homo sapiens
<400> 45
   cgtggcactt acattccag 19
<210> 46
   <211> 19
   <212> DNA
   <213> Homo sapiens
<400> 46
   ggagtggatg tgcaaaatg 19
<210> 47
   <211> 21
   <212> DNA
   <213> Homo sapiens
<400> 47
   ggctgtataa gccagcatca t 21
<210> 48
   <211> 21
   <212> DNA
   <213> Homo sapiens
<400> 48
   cccattctgt gcatgtcttt t 21
<210> 49
   <211> 21
   <212> DNA
   <213> Homo sapiens
<400> 49
   aacaaagcag ccaccgcaga c 21
<210> 50
   <211> 21
   <212> DNA
   <213> Homo sapiens
<400> 50
   cctgacctta cttcacagcc t 21
<210> 51
   <211> 18
   <212> DNA
   <213> Homo sapiens
<400> 51
   tttggaactt ggctcttg 18
<210> 52
   <211> 32
   <212> DNA
   <213> Homo sapiens
<400> 52
   cagacagcag ctgacagtcc aagaacagga ct 32
<210> 53
   <211> 20
   <212> DNA
   <213> Homo sapiens
<400> 53
   gccacgtgat tcttccacaa 20
<210> 54
   <211> 19
   <212> DNA
   <213> Homo sapiens
<400> 54
   ctgtctgggc atccaggat 19
<210> 55
   <211> 22
   <212> DNA
   <213> Homo sapiens
<400> 55
   agatccagct caggtcggta cc 22
<210> 56
   <211> 20
   <212> DNA
   <213> Homo sapiens
<400> 56
   ccggagcttt cagaattgac 20
<210> 57
   <211> 21
   <212> DNA
   <213> Homo sapiens
<400> 57
   ttgttctctg cagcagcaaa c 21
<210> 58
   <211> 22
   <212> DNA
   <213> Homo sapiens
<400> 58
   ccgacaactt tctcttcagg tc 22
<210> 59
   <211> 21
   <212> DNA
   <213> Homo sapiens
<400> 59
   ctggtagtta gacttggtct c 21
<210> 60
   <211> 20
   <212> DNA
   <213> Homo sapiens
<400> 60
   gccaatgagg tagctggtgg 20